# EUROPEAN PATENT SPECIFICATION

(11) **EP 2 936 163 B1**
(45) Date of publication and mention of the grant of the patent: **17.06.2020**
(21) Application number: 13821708.8
(22) Date of filing: 18.12.2013
(51) Int. Cl.: G01N 33/68

(54) **DETECTION OF MEMBRANE PROTEINS**
NACHWEIS VON MEMBRANPROTEINEN
DÉTECTION DES PROTÉINES MEMBRANAIRES

(30) Priority: 18.12.2012 GB 201222833
(43) Date of publication of application: 28.10.2015
(73) Proprietor: Oxford University Innovation Limited, Botley Oxford OX2 0JB (GB)
(72) Inventor: LAGANOWSKY, Arthur, Oxford Oxfordshire OX1 3QZ (GB); READING, Eamonn, Oxford Oxfordshire OX1 3QZ (GB)
(74) Representative: Mathys & Squire
(86) International application number: PCT/GB2013/053345
(87) International publication number: WO 2014/096821

(56) References cited:
- WO-A1-2012/172378
- HANSEN R K ET AL: "Hydrogen/deuterium exchange of hydrophobic peptides in model membranes by electrospray ionization mass spectrometry", JOURNAL OF THE AMERICAN SOCIETY FOR MASS SPECTROMETRY, ELSEVIER SCIENCE INC, US, vol. 13, no. 12, 1 December 2002 (2002-12-01), pages 1376-1387, XP027414746, ISSN: 1044-0305 [retrieved on 2002-12-01]
- NELSON P BARRERA ET AL: "Mass spectrometry of membrane transporters reveals subunit stoichiometry and interactions", NATURE METHODS, vol. 6, no. 8, 5 July 2009 (2009-07-05), pages 585-587, XP055034141, ISSN: 1548-7091, DOI: 10.1038/nmeth.1347
- SEDDON A M ET AL: "Membrane proteins, lipids and detergents: not just a soap opera", BIOCHIMICA ET BIOPHYSICA ACTA (BBA) - BIOMEMBR, ELSEVIER, AMSTERDAM, NL, vol. 1666, no. 1-2, 3 November 2004 (2004-11-03), pages 105-117, XP004617558, ISSN: 0005-2736, DOI: 10.1016/J.BBAMEM.2004.04.011
- KALIPATNAPU SHANTI ET AL: "Membrane protein solubilization: recent advances and challenges in solubilization of serotonin1A receptors.", IUBMB LIFE JUL 2005, vol. 57, no. 7, July 2005 (2005-07), pages 505-512, ISSN: 1521-6543
- LE MAIRE^A M ET AL: "Interaction of membrane proteins and lipids with solubilizing detergents", BIOCHIMICA ET BIOPHYSICA ACTA (BBA) - BIOMEMBR, ELSEVIER, AMSTERDAM, NL, vol. 1508, no. 1-2, 23 November 2000 (2000-11-23), pages 86-111, XP004273339, ISSN: 0005-2736, DOI: 10.1016/S0304-4157(00)00010-1

## Description

The work leading to this invention has received funding from the European Research Council under the European Union's Seventh Framework Programme (FP7/2007-2013/ERC grant agreement no. 268851).

### Field of the Invention

The present invention relates to the detection of membrane proteins. More particularly, the present invention relates to methods for the detection of membrane protein complexes by mass spectrometry. The methods disclosed herein may be used to detect membrane proteins, including intact membrane protein complexes and complexes of membrane proteins with ligands such as therapeutic agents.

### Background to the Invention

Membrane proteins are responsible for a wide range of biological functions. Some of the most prevalent human diseases, including some cancers, result from their dysfunction. Despite representing around a third of the human genome, membrane proteins represent targets for more than half of all current therapeutic agents. As a significant biological target in disease and cancer, their study by traditional structural biology approaches, such as X-ray crystallography and nuclear magnetic resonance, has been frustrated by limitations relating to their expression and solubility. Furthermore, X-ray analysis, in the majority of cases, has been limited by crystallographic resolution hindering the assignment of bound moieties.

In contrast to classical structural biology methods, mass spectrometry (MS) of intact complexes, sometimes referred to as "native MS", is a rapid and sensitive technique that can provide invaluable information on protein complexes, such as specifically bound small molecules. MS of intact soluble membrane proteins has emerged as a powerful technique to study the stoichiometry, structure-function and dynamics of protein assemblies. Recent developments have extended this technique to the study of membrane protein complexes, where it has already revealed subunit stoichiometries and specific phospholipid interactions. Consequently, MS of intact membrane protein complexes is set to become an indispensable tool for membrane protein biochemical and structural studies.

Membrane protein MS is normally performed using a detergent micelle in which the protein is contained. The membrane protein may be prepared by buffer exchange of the purified protein into an MS-compatible buffer supplemented with the detergent. The micellar solution is then ionized by means of nanoelectrospray and transmission into the mass spectrometer. Non-ionic detergents such as n-dodecyl-β-D-maltoside (DDM) are typically used to form these micellar solutions, as high concentrations of non-ionic detergents can be tolerated more readily during the electrospray process.

In order to obtain well-resolved charge state distributions that enable mass measurements, removal of the detergent through thermal activation is required. This is normally achieved by increasing collisional activation in the mass spectrometer, which results in detergent removal and release of the intact ionized protein assembly. However, collisional activation not only liberates membrane protein complexes from detergent micelles, but it can also result in the dissociation of the protein complexes. Activation can lead to local structural unfolding and, upon further activation, a threshold is reached whereby an unfolded protein subunit is ejected from the protein complex. This may not be preferable from the point of view of detecting intact protein complexes or measuring the structural topology of protein complexes.

There exists a need for improved MS methods for detecting membrane proteins which minimise structural unfolding and dissociation of the proteins during detection. In particular, there exists a need for MS methods which avoid the relatively high activation energy requirements normally needed to study membrane proteins by MS.

Hansen et al. (J. Am. Soc. Mass. Spectrom., 2002, 13(12), 1376-1387) describe the hydrogen/deuterium exchange properties of hydrophobic peptides in model membranes by electrospray ionisation mass spectrometry.

### Summary of the Invention

The present invention is based, at least in part, on a surprising discovery that by using polyoxyalkylene glycol detergents to form micellar solutions of membrane proteins, the charge state of the membrane proteins may be significantly reduced. This is turn means that the activation energy needed to liberate the membrane protein from the detergent micelle during detection is lowered. Thus, for instance, micellar solutions formed using polyoxyalkylene glycol detergents may be used to analyse membrane proteins, and their interactions with ligands, in a substantially intact, native-like state.

According to the present invention there is provided a method of detecting a membrane protein by mass spectrometry, wherein the method comprises:
(a) providing a solution comprising a detergent micelle in which said membrane protein is contained, wherein said solution contains a polyoxyalkylene glycol detergent having a molecular weight of at least 300 Da;
(b) providing a mass spectrometer comprising a nanoelectrospray ionisation source, a mass analyser and a detector;
(c) vaporising the solution using the nanoelectrospray ionisation source under conditions such that the membrane protein is released from the micelle;
(d) ionising the membrane protein;
(e) resolving the ionised membrane protein using the mass analyser; and
(f) detecting the resolved membrane protein using the detector.

Also provided is the use of a solution comprising a detergent micelle in which a complex is contained, wherein the complex comprises a membrane protein bound to one or more ligands (*e.g*. bound to a therapeutic agent), and wherein the solution comprises a polyoxyalkylene glycol detergent having a molecular weight of at least 300 Da, for the detection of said complex by mass spectrometry.

### Description of the Drawings

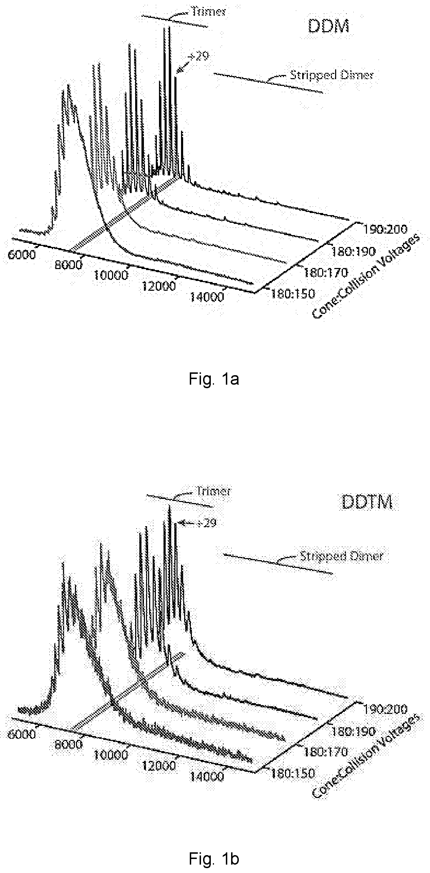

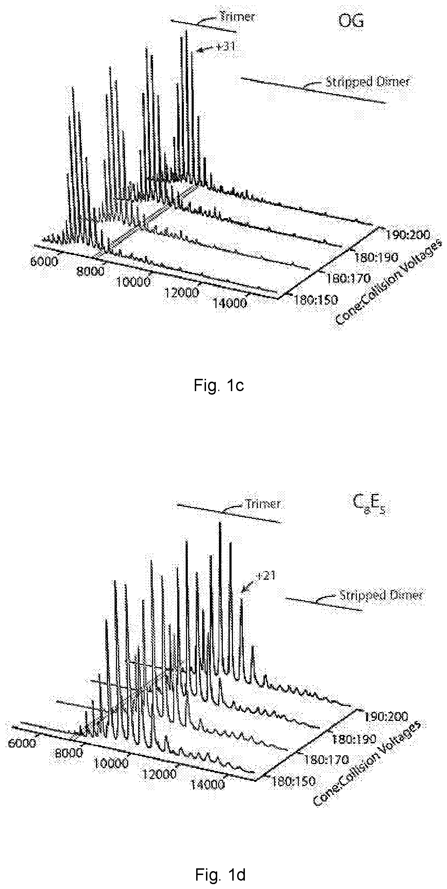

Figure 1 depicts a mass spectra activation series for selected detergents on *E. coli* ammonium channel C-terminally fused to green fluorescent protein (AmtB-GFP). Mass spectra are presented for the following detergents: (a) AmtB-GFP exchanged into n-dodecyl-β-D-maltoside (DDM), which served as the control in this experiment; (b) n-dodecyl-β-D-thiomaltopyranoside (DDTM); (c) n-octyl-β-D-glucopyranoside (OG); and (d) C₈E₅, a polyoxyethylene glycol detergent. The series highlights the activation energy required to achieve resolved peaks. The trimer and stripped dimer regions of the mass spectrum are shown as a function of activation energy, i.e. increasing cone and collision voltages. The shaded block region highlights the position of the peak for the +29 charge state of the AmtB-GFP trimeric complex. Charge states are labelled for the highest activation series.

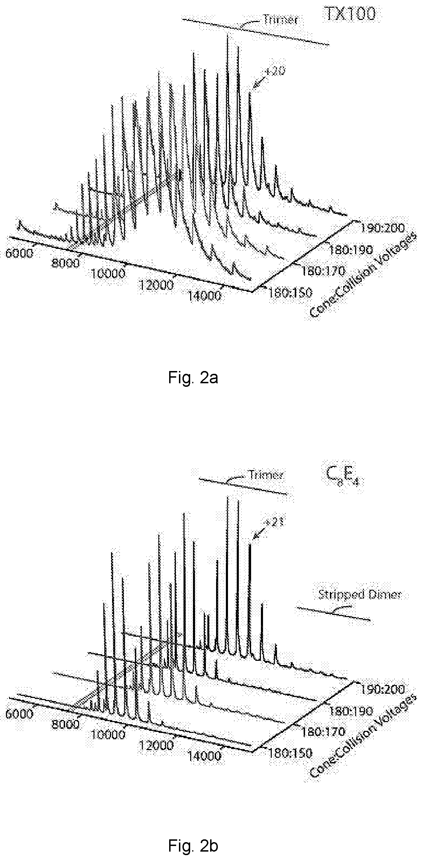

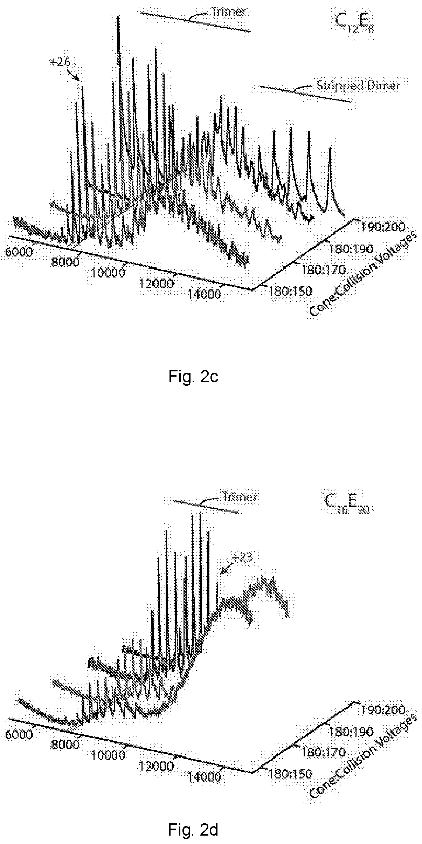

Figure 2 shows a mass spectra activation series for AmtB-GFP detergent exchanged into various polyoxyalkylene glycol detergents. Mass spectra are presented in respect of: (a) Triton X-100 (TX100); (b) C₈E₄; (c) C₁₂E₈; and (d) C₁₆E₂₀ detergents. Mass spectra are shown and labelled as described in Figure 1.

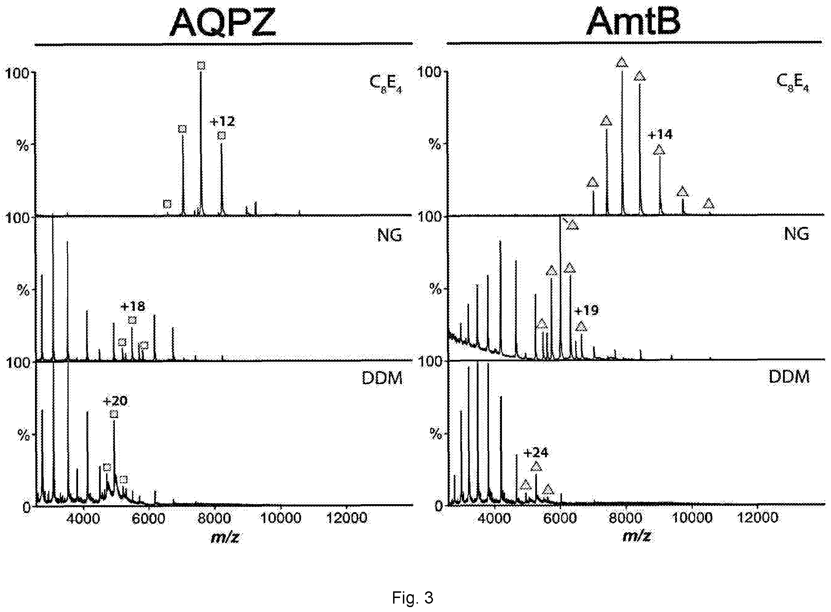

Figure 3 shows a comparison of mass spectra for Aquaporin Z (AQPZ) and ammonium channel (AmtB) membrane protein complexes in three detergents: polyoxyethylene glycol C₈E₄ (C₈E₄), n-nonyl-β-D-glucopyranoside (NG), and n-dodecyl-β-D-maltoside (DDM). Peaks corresponding to tetrameric AQPZ (left panel) and trimeric AmtB (right panel) are annotated with squares and triangles, respectively. Charge states are labelled for one peak in the charge state distribution.

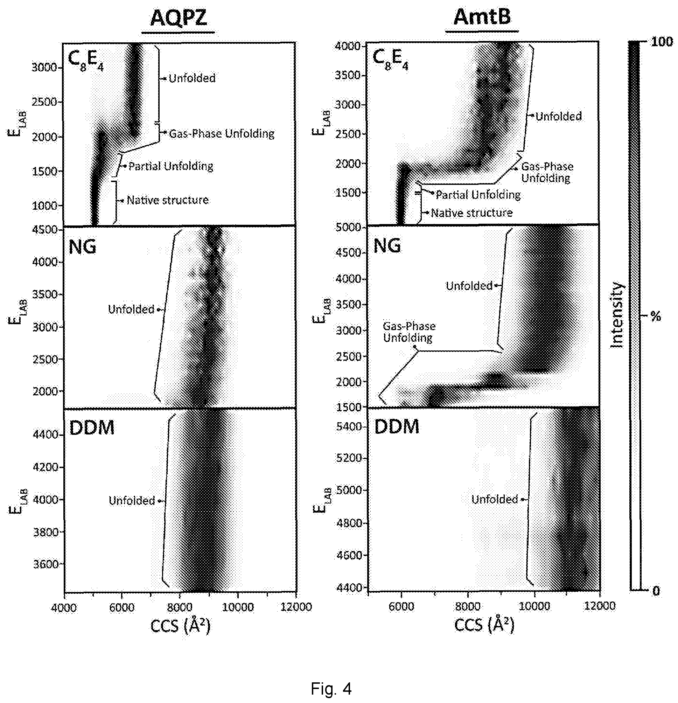

Figure 4 shows ion mobility mass spectrometry of AQPZ and AmtB membrane protein complexes in the following detergents: polyoxyethylene glycol C₈E₄ (C₈E₄), n-nonyl-β-D-glucopyranoside (NG), and n-dodecyl-β-D-maltoside (DDM). Provided in the inset of each plot is the detergent that the protein complex is contained within. Shown are charge states +13, +19, and +19 for AQPZ (left panel) and +17, +21, and +23 for AmtB (right panel) in C₈E₄, NG, and DDM, respectively. Specifically, odd charge states were chosen to avoid overlap with activated species as is possible for even charge states. Shown are collision induced unfolding (CIU) fingerprints. These plots show the experimental collisional cross section (CCS) as a function of E_{LAB} (laboratory frame energy, which is the collision voltage multiplied by charge state) at a resolution of 5 collision volts.

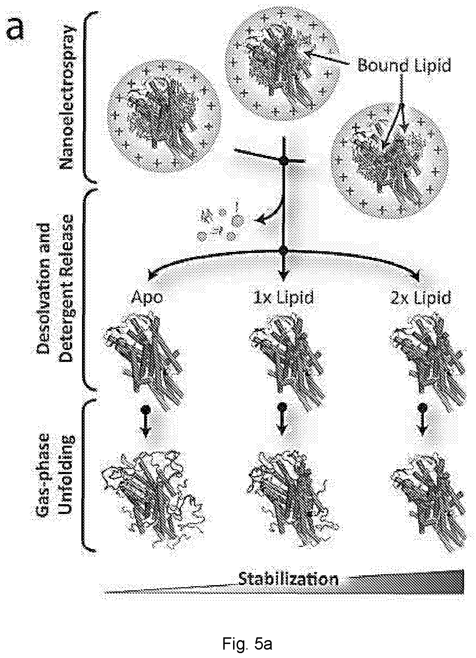

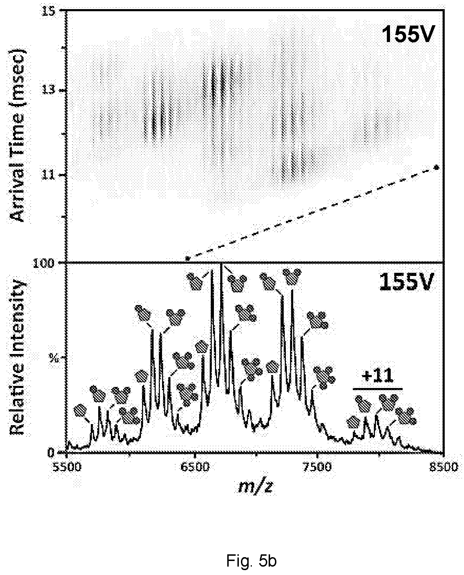

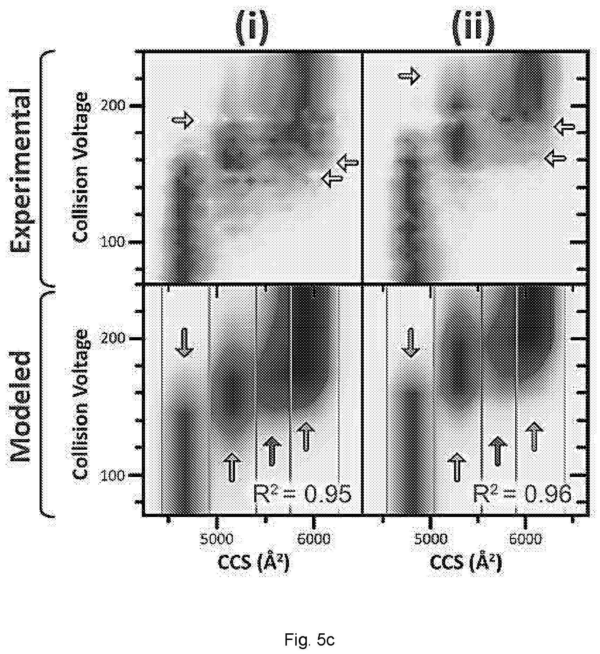

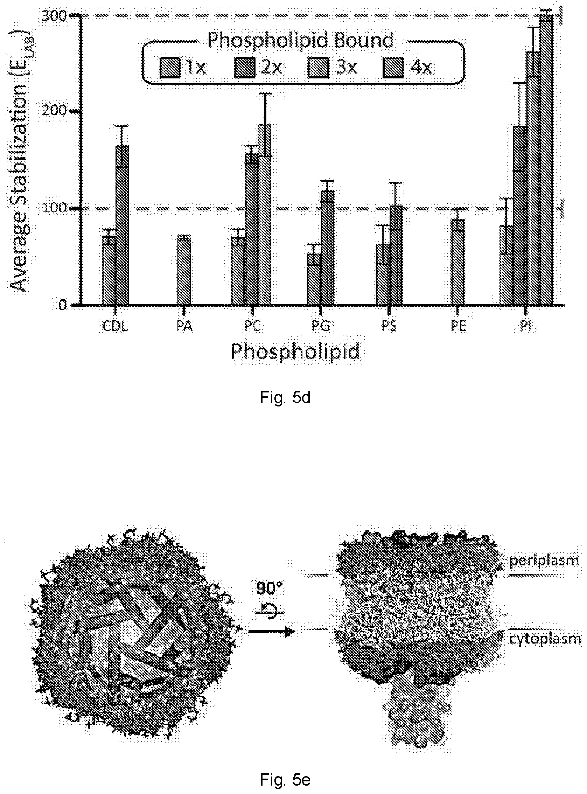

Figure 5 illustrates the resistance of the mechanosensitive channel of large conductance (MscL) to unfolding in the presence of phospholipids. Figure 5a provides a schematic representation of the experiment designed to measure resistance to unfolding in the gas phase. The membrane protein complex, in this case MscL, is shown in the electrospray droplet, within a detergent micelle, with lipids bound. Following desolvation, and activation to remove detergent, the naked gas phase complex is maintained close to its native state prior to gas phase unfolding. Figure 5b shows mass spectra and ion mobility of MscL bound to phosphatidylinositol (PI) molecules. Charge states in the mass spectra (+11 to +15) contain multiple peaks, assigned to different lipid bound states. At <155 V collision energy arrival time distributions observed for the +12 charge state correspond to those calculated for near native states intermediate and extended species. Note the stronger intensity for near native and intermediate species when bound to PI as opposed to the apo form of the complex. Figure 5c shows a plot of collision voltage against CCS for +12 charge state of *apo* (i) and MscL bound to four PI (ii). Experimental and modelled unfolding plots are shown (upper and lower plots respectively). Differences are observed in collision voltages that bring about the different transitions with the most extended species (CCS = -6000 Å²) stabilised to the greatest extent. The collision voltages (horizontal arrows) at which transitions occur and CCS values (vertical arrows) are derived from modelling. *R²* values are shown in the modelled data. Figure 5d shows the average stabilisation, calculated from the parameters defined by fitting, for the +12 charge state of MscL bound to different phospholipids. Reported are the average and SEM (*N*=*3*)*.* Phospholipids abbreviations are shown. Figure 5e shows MD constrained by CCS measurements results in POPC molecules forming an annular belt around MscL. POPC lipid side chains are shown in stick representation and headgroups.

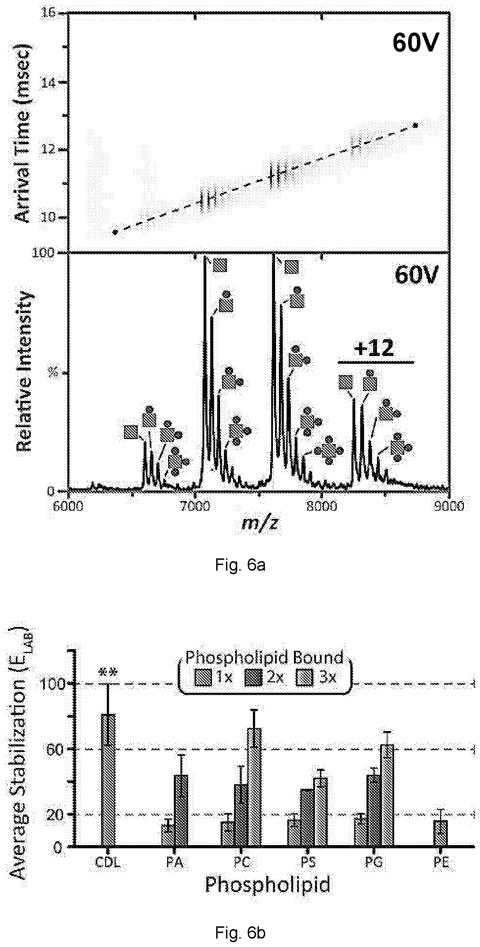

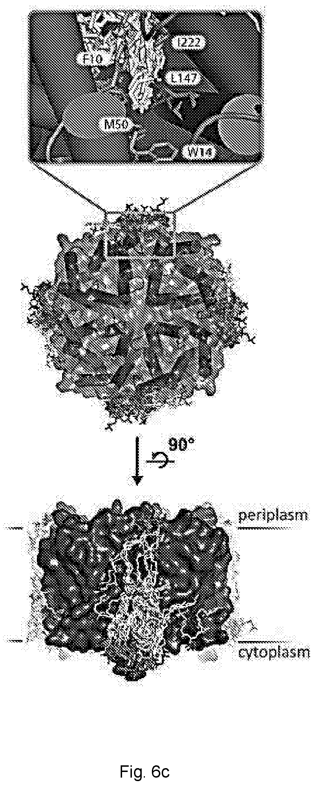

Figure 6 shows that AQPZ is stabilised indiscriminately by multiple phospholipids with the exception of cardiolipin. Figure 6a shows mass spectra which reveal one to six bound phosphatidylcholine molecules resolved at a collision voltage (CV) of 130 V. Ion mobility mass spectra are shown in linear scale overlaid with calculated arrival times for the native state (white dotted line). For all charge states (+12 to +15) the arrival times for apo and lipid bound forms of AQPZ agree with the CCS value calculated from the crystal structure at low CV. Figure 6b shows the average stabilisation for the +13 charge state of AQPZ titrated with different phospholipid molecules calculated from unfolding parameters as in Figure 5c. One-way ANOVA (*N* = 3): ***P* < 0.01. Figure 6c shows that molecular dynamics coupled with CCS measurements illuminate probable single POPC binding sites for AQPZ. Shown are side and top views. PC lipid side chains are shown as in Figure 5e and found to cluster to the interfacial regions of protein subunits in which tails of POPC molecules penetrate a hydrophobic pocket (inset).

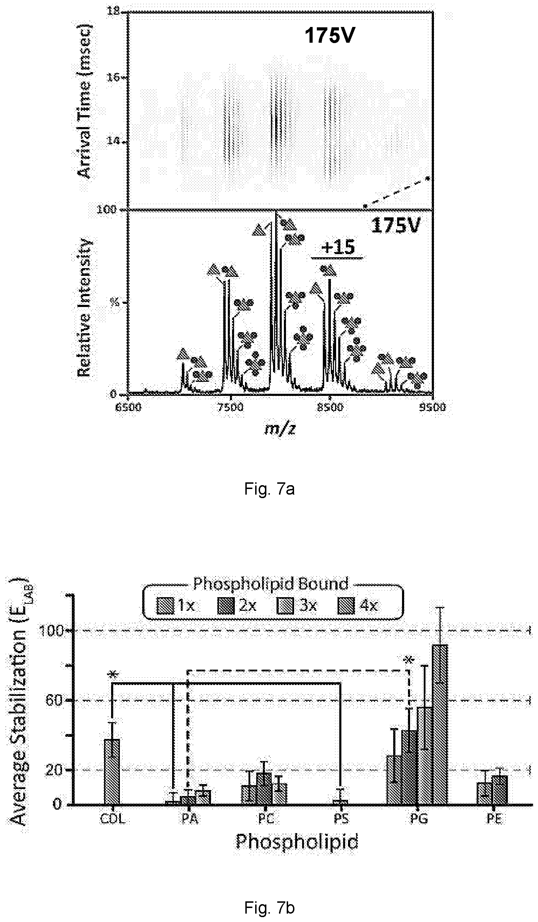

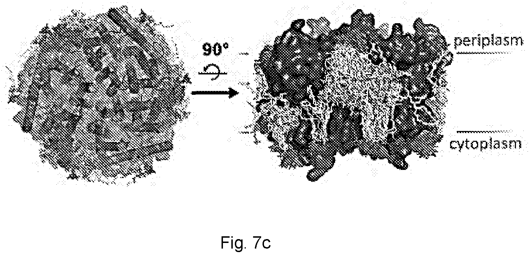

Figure 7 illustrates that phospholipid binding to the Ammonia channel (AmtB) results in a range of stabilising effects. Figure 7a shows ion mobility mass spectra of AmtB bound to one to four PG molecules at moderate collision activation (175 V). Arrival times for the various charge states at 175 V reveal non-native, trimeric structures. Figure 7b shows average stabilisation of AmtB bound to different phospholipids for the +15 charge state determined through gas-phase unfolding analyses reveals that AmtB is weakly stabilised by the majority of phospholipids. Results are shown as in Figure 6b with **P* < 0.05. CDL and PG are the exception with significant stabilisation conferred by binding, and additive stability for binding additional PG molecules. Figure 7c shows MD filtered by CCS measurements localized POPC lipids to shallow grooves between subunits, as well as to other probable locations on individual monomers.

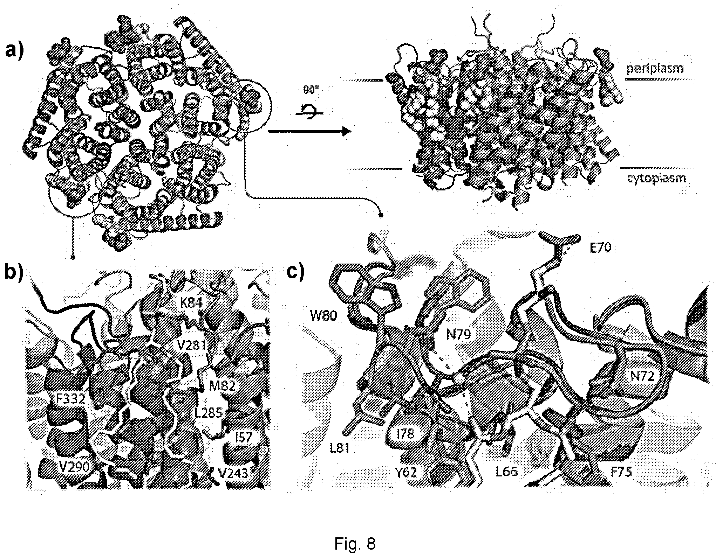

Figure 8 shows the crystal structure of AmtB bound to PG. Figure 8a shows the structure viewed from the periplasm and perpendicular to the membrane plane. AmtB is shown in ribbon representation. PG is shown as spheres and shaded as in Figure 5e. Resolved lipid molecules are oriented with headgroups on the periplasmic side, positioning these lipids in the outer leaflet of the inner membrane. PG molecules are bound at subunit interfaces and at a specific site within each monomer. Figure 8b shows the molecular interactions of PG located in the subunit interface. Residue labels are shown. Hydrophobic residues in contact with the lipid tails are shown in stick representation. Hydrogen bonds are formed between the phospho headgroup and K84, and a water (aqua sphere) bridge. Figure 8c depicts a distinct conformational change in a loop region, namely residues 70-81, induced by binding PG. Shown is the comparison of AmtB structure (PDB 1U7G) with AmtB bound to PG. The arrows highlight the shift in specific residues to the PG-bound conformation.

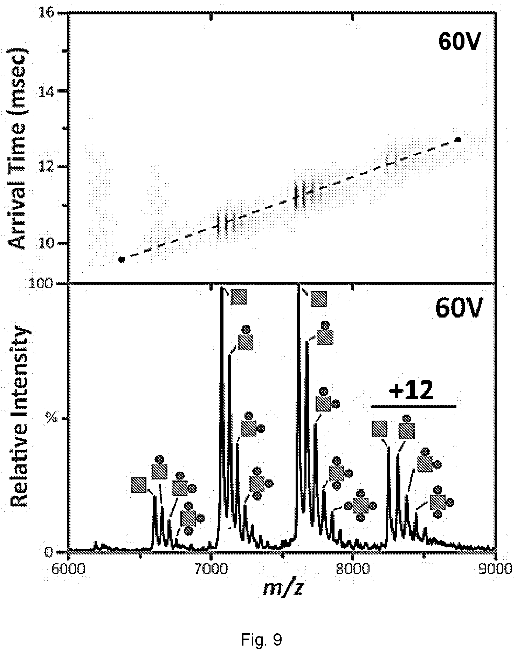

Figure 9 shows mass spectra and ion mobility analysis of AQPZ tetramer from *Escherichia coli* bound to up to four phosphatidylcholine phospholipid molecules revealing well resolved peaks in both dimensions, as well as the achievement of native-like state in the gas phase. The white dashed line represents the collision cross section calculated for the crystal structure.

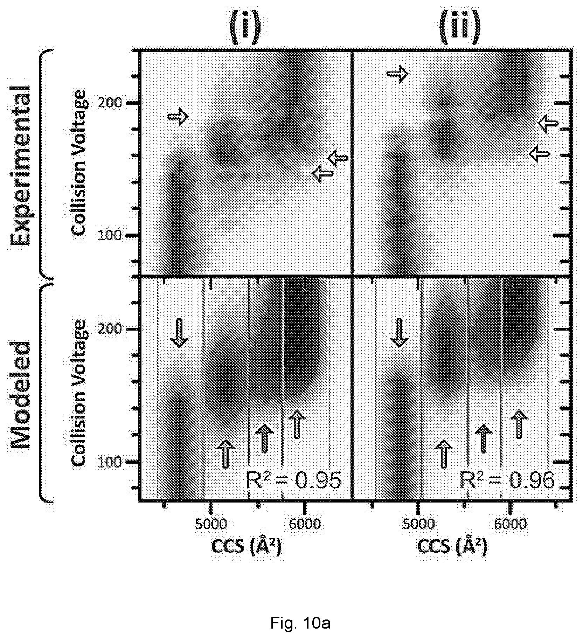

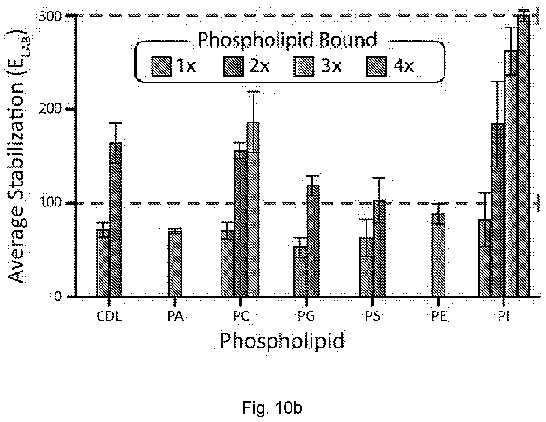

Figure 10a shows a four state gas-phase unfolding series of the pentameric MscL in its apo (i) and phosphatidylinositol (PI) (ii) bound state. Experimental data are shown in the top insets with the modelled unfolding series in the bottom insets. Figure 10b shows average stabilisation of the MscL protein complex bound to different phospholipid forms and numbers reveals differences in protein complex stabilisation upon small molecule binding.

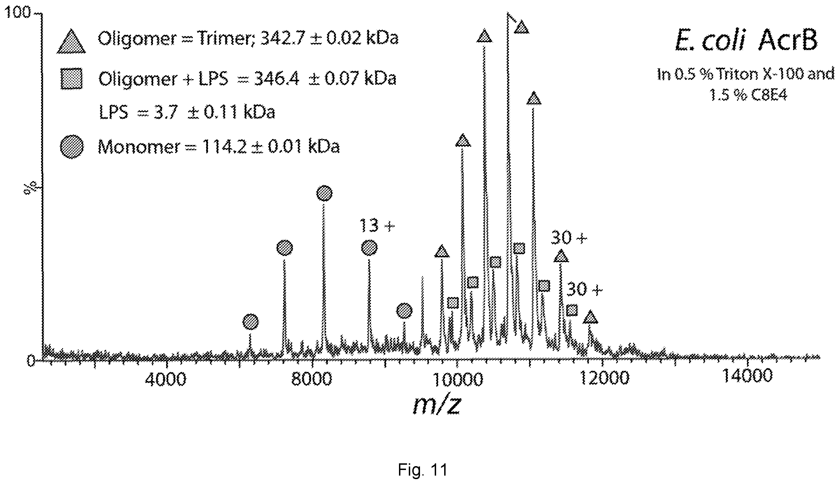

Figure 11 shows mass spectra of intact Acriflavine resistance protein B (AcrB) from *Escherichia coli.* Spectra were collected from a 200 mM ammonium acetate solution pH 8.0, containing a detergent mixture of 0.5 % Triton X-100 and 1.5 % C₈E₄.

### Description of Various Embodiments

The present invention provides a method of detecting a membrane protein by mass spectrometry. A method of the present invention involves the use of a solution comprising a detergent micelle in which the membrane protein is contained, wherein said solution contains a polyoxyalkylene glycol detergent. The solution is vaporised using a nanoelectrospray ionisation source under conditions such that the membrane protein is released from the detergent micelle. The membrane protein is ionised, and subsequently resolved and detected. The method of the present invention may be particularly advantageous for detecting membrane protein complexes in a folded state.

Membrane proteins can be grouped into integral membrane proteins and peripheral membrane proteins. Integral membrane proteins may have one or more segments embedded within a membrane and may be bound to the lipid bilayer. Peripheral membrane proteins may be temporarily associated with the lipid bilayer and/or integral membrane proteins. In an embodiment, the membrane protein is an integral membrane protein.

Membrane proteins may be composed of one (mono) or more (multi) associated polypeptide chains. Thus, the membrane protein may be a monomeric or a multimeric membrane protein, for example an oligomeric membrane protein. Oligomeric membrane proteins include both homooligomeric (identical polypeptide chains) and heterooligomeric (different polypeptide chains) proteins.

In an embodiment, the membrane protein is an integral membrane protein selected from G protein-coupled receptors (GPCRs), membrane transporters, membrane channels, ATP-binding cassette transporters (ABC-transporters) and proton driven transporters. In an embodiment, the membrane protein is selected from EmrE, LmrP, MscL, BtuCD, BtuC₂D₂, LmrCD, MacB, MexB, P-gp, MsbA, peptide transporters, NorM, KirBac3.1, AQPZ or AmtB. In a particular embodiment, the membrane protein is AQPZ or AmtB.

In an embodiment, the membrane protein has a molecular weight of from about 10³ Daltons to about 10¹² Daltons, *e.g*. from about 10³ Daltons to about 10⁶ Daltons.

Methods for the purification and expression of membrane proteins are known in the art. By way of example, Barrera et al, Nat. Methods 2009, 6, 585-587 describe methods for the purification of MacB, LmrCD, and EmrE. Moreover, Drew et al, Nat. Protoc., 2008, 3, 784-798 describe a GFP fusion construct methodology in which yields in the overexpression and purification of membrane proteins are improved, while Aller et al, Science, 2009, 323, 1718-1722 describe P-glycoprotein expression and purification. In order to produce high quality mass spectra, the membrane protein should preferably be relatively pure and homogenous, equivalent to crystallographic-grade material.

The membrane protein may be in the form of a complex with a ligand. Thus, for example, the membrane protein may be in the form of a complex with one or more ligands selected from therapeutic agents, lipids and nucleosides.

In one embodiment, the membrane protein is in the form of a complex with one or more therapeutic agents. In this regard, the present methods may be used to detect binding between a membrane protein and a therapeutic agent. In particular, a method of the present invention may allow one or more structural characteristics (*e.g*. stoichiometry) of a membrane protein-therapeutic agent complex to be determined, and/or may also be used to detect conformational changes that take place upon binding of a therapeutic agent to the membrane protein.

A method of the present invention may allow therapeutic agents to be screened. In contrast to indirect methods such as fluorescence or calorimetry, the present method may allow therapeutic agents to be screened directly. In particular, a method may be used to screen for the binding of activators and transporter substrates which are difficult to screen using conventional *in vivo* methodologies. Moreover, unlike X-ray crystallography, the present methods are not complicated by the inherent structural flexibility of membrane protein-therapeutic agent complexes and may allow the dynamical behaviour of membrane proteins and their interaction with therapeutic agents to be studied.

The therapeutic agent may be an active compound which, when administered to an organism (human or non-human animal), induces a desired pharmacologic, immunogenic, and/or physiologic effect by local and/or systemic action. Examples of therapeutic agents include, without limitation, drugs, vaccines and biopharmaceutical agents. Thus, therapeutic agents may include small molecule drugs, therapeutic proteins, peptides and fragments thereof (whether naturally occurring, chemically synthesised or recombinantly produced), and nucleic acid molecules (including both double-and single-stranded molecules, gene constructs, expression vectors, antisense molecules and the like). Therapeutic agents may also include substrates, inhibitors, activators, neurotransmitters, agonists and antagonists. The therapeutic agent may be a synthetic or naturally occurring compound. The therapeutic agent may be a drug candidate or other agent suspected of having therapeutic application.

Particular examples of therapeutic agents include, but are not limited to, anti-cancer agents, anti-infective agents (e.g. antibiotics and antiviral agents), analgesic agents, anorexic agents, anti-inflammatory agents, antiepileptic agents, anaesthetic agents, hypnotic agents, sedatives, antipsychotic agents, neuroleptic agents, antidepressants, anxiolytics, antagonists, neuron blocking agents, anticholinergic and cholinomimetic agents, antimuscarinic and muscarinic agents, antiadrenergics agents, hormones, nutrients, antiarthritics agents, antiasthmatic agents, anticonvulsants, antihistamines, antinauseants agents, antineoplastic agents, antipruritics agents, antipyretic agents; antispasmodic agents, cardiovascular agents (*e.g*. calcium channel blockers, beta-blockers, beta-agonists, antiarrhythmic agents, antihypertensive agents, diuretics and vasodilators), central nervous system stimulants; decongestants, hormones, bone growth stimulants, bone resorption inhibitors, immunosuppressive agents, muscle relaxants, psychostimulants, sedatives and tranquilisers. It will be appreciated that this list of therapeutic agents is merely illustrative and should not be considered to be limiting. Many other therapeutic agents are known in the art and may be utilised in a method of the present invention. A detailed description of various therapeutic agents may be found in *e.g*. Remington's Pharmaceutical Sciences (21st edition, 2005, Mack Publishing Company). The therapeutic agent may exhibit optical isomerism and/or diastereoisomerism. Accordingly, the therapeutic agent may be in the form of a single enantiomer or diastereoisomer, or a mixture (*e*.*g*. a racemic mixture) thereof.

In an embodiment, the therapeutic agent has a molecular weight of less than 2000 Daltons, *e.g*. less than 1500 Daltons, *e.g*. less than 1000 Daltons, *e.g*. less than 500 Daltons. In an embodiment, the therapeutic agent is a non-polymeric organic compound having a molecular weight of less than 1000 Daltons, *e.g*. less than 800 Daltons, *e.g*. less than 500 Daltons.

In an embodiment, the therapeutic agent is an inhibitor or an activator, e.g. an activator or inhibitor of the membrane protein to which it is bound. In a particular embodiment, the therapeutic agent is a cyclic peptide inhibitor. Examples of cyclic peptide inhibitors include QZ59Se and HT-55. Examples of activators include HT-44 and HT-122.

In an embodiment, the therapeutic agent is an anti-cancer agent. In a particular embodiment, the therapeutic agent is selected from doxorubicin, digoxin, loperamide, berberine, irinotecan, vinblastine, paclitaxel, fexofenadine, cyclosporin, quinidine, tariquidar and verapamil. In an embodiment, the therapeutic agent is cyclosporin A.

Binding of the therapeutic agent to the membrane protein may be via a non-covalent or a covalent interaction. In particular, binding of the therapeutic agent to the membrane protein may be via intermolecular forces such as ionic bonds, hydrogen bonds and van der Waals forces. Binding of the therapeutic agent to the membrane protein may be reversible or irreversible. In an embodiment, the therapeutic agent is bound to the membrane protein via a reversible bond.

The membrane protein-therapeutic agent complex may comprise one or more other components. In one embodiment, the complex further comprises a lipid. Thus, for instance, a method of the present invention may be used to determine whether the presence of a lipid affects binding of the therapeutic agent to the membrane protein. In an embodiment, the complex comprises a nucleotide. In an embodiment, the complex comprises a lipid and a nucleotide.

The present method involves the use of a micellar solution which comprises a detergent micelle in which the membrane protein is contained. The membrane protein is encapsulated in a micelle for solubilisation, which may at least partially shield the protein during the electrospray ionisation process. Without wishing to be bound by theory, it is believed that the micelle may shield the protein during the droplet phase of the electrospray ionisation process and, moreover, may afford at least partial shielding from ionisation of the protein during this process. The detergent micelle may exert a pressure sufficient to maintain the structure of the membrane protein, thereby minimising the deleterious effects associated with vaporisation and substantially retaining interactions between the membrane protein and any ligand and interactions within any subunits of the membrane protein. The solution will typically comprise a plurality of micelles containing the membrane protein. The micellar solution may be formed by *e.g.* incubating the membrane protein in the presence of the detergent.

Preferably, the membrane protein is maintained in the detergent micelle in an intact, folded state. This may allow the membrane protein to be detected in its "native" state. Alternatively, the membrane protein may be present within the detergent micelle in a partially folded or unfolded state.

According to the present invention, the micellar solution comprises a polyoxyalkylene glycol detergent having a molecular weight of at least 300 Da. Polyoxyalkylene glycol detergents are generally non-ionic detergents and comprise an optionally substituted polyoxyalkylene glycol chain. In an embodiment, the polyoxyalkylene glycol chain is formed of one or more monomers, wherein said one or more monomers include ethylene glycol monomers and/or propylene glycol monomers. Preferably, the polyoxyalkylene glycol chain which is etherified at one end by *e.g.* a hydrocarbyl group selected from C₁₋₃₀ alkyl, C₂₋₃₀ alkenyl, C₂₋₃₀ alkynyl, C₃₋₃₀ carbocyclyl, C₇₋₃₀ aralkyl and C₇₋₃₀ alkaryl. In an embodiment, the polyoxyalkylene glycol detergent has a molecular weight of from about 300 Da to 2000 Da, *e.g.* from 300 Da to 1000 Da, *e.g.* from 300 Da to 600 Da or from 500 Da to 1000 Da.

In a preferred embodiment, the polyoxyalkylene glycol detergent is a polyoxyethylene glycol detergent. Polyoxyethylene glycol detergents are generally non-ionic detergents and comprise an optionally substituted polyoxyethylene glycol chain. Preferably, the polyoxyethylene glycol detergent is formed of a polyoxyethylene glycol which is etherified at one end by *e.g.* a hydrocarbyl group selected from C₁₋₃₀ alkyl, C₂₋₃₀ alkenyl, C₂₋₃₀ alkynyl, C₃₋₃₀ carbocyclyl, C₇₋₃₀ aralkyl and C₇₋₃₀ alkaryl. In an embodiment, the polyoxyethylene glycol detergent has a molecular weight of from 300 Da to 2000 Da, *e.g*. from 300 Da to 1000 Da, *e.g*. from 300 Da to 600 Da or from 500 Da to 1000 Da. Preferred detergents include, without limitation, tetraethylene glycol monooctyl ether ("C₈E₄"), pentaethylene glycol monooctyl ether ("C₈E₅"), pentaethylene glycol monodecyl ether ("C₁₀E₅"), polyoxyethylene (8) dodecyl ether ("C₁₂E₈"), polyoxyethylene (20) cetyl ether ("C₁₆E₂₀") and polyethylene glycol *tert*-octylphenyl ether (*t*-oct-C₆H₄-(OCH₂CH₂)ₓOH, where x is 9-10; also known as "Triton X-100").

The micellar solution may be formed using a single polyoxyalkylene glycol detergent or a mixture of different polyoxyalkylene glycol detergents. The micellar solution may contain one or more detergents in addition to the polyoxyalkylene glycol detergent. Examples of suitable detergents include non-ionic detergents such as *n*-dodecyl-β-D-maltoside, nonylglucoside, glycosides, neopentyl glycols, facade EM, maltosides, glucosides, and mixtures thereof. In an embodiment, the micellar solution does not contain *n*-dodecyl β-D-maltoside. In an embodiment, the micellar solution does not contain any detergents apart from polyoxyalkylene glycol detergent(s).

In order to minimise dissociation of the membrane protein or precipitation of the protein, the detergent should be present in the micellar solution at a concentration at least equal to, and preferably at two times the critical micelle concentration (CMC) of the detergent. The critical micelle concentration of the detergent may be determined experimentally using methods known in the art, or it may be obtained from *e.g*. a textbook, product catalogue or website. For example, the Anatrace products catalogue provides CMC data. Typically, critical micelle concentration values are determined in water at 25 °C. Thus, in an embodiment, the solution is an aqueous solution and the detergent is present in the solution at a concentration at least equal to, and preferably greater than, the critical micelle concentration of the detergent in water at 25 °C. The detergent is preferably present at a concentration at least twice than that of the critical micelle concentration of the detergent in water at 25 °C.

In an embodiment, the detergent is present in the micellar solution at a concentration of from about 100 µM to about 100 mM, *e*.*g*. from about 100 µM to about 200 µM. In an embodiment, the membrane protein is present in the micellar solution at a concentration of from about 1 nM to about 1 mM, *e.g*. from about 800 nM to about 900 nM.

In an embodiment, the molar ratio of the detergent to the membrane protein is from about 10:1 to 150:1, *e.g*. from about 30:1 to about 125:1, *e.g*. from about 50:1 to about 100:1. In a preferred embodiment, the molar ratio of the detergent to the membrane protein is less than or equal to 100:1.

Where the membrane protein is in the form of a complex with a therapeutic agent, the micellar solution preferably comprises a molar excess of the therapeutic agent as compared to the membrane protein. In an embodiment, the molar ratio of the therapeutic agent to the membrane protein is at least 2:1, *e.g*. at least 5:1, *e.g.* at least 10:1. In an embodiment, the therapeutic agent is present in the micellar solution at a concentration of at least 100 nM, *e*.*g*. from 100 nM to 900 nM.

The micellar solution may comprise one or more other components. In particular, the micellar solution preferably contains a buffer. Ammonium acetate is particularly preferred in this regard. The concentration of ammonium acetate is preferably at least 200 micromolar. Preferably the pH of the buffer is in the range of from about 5 to about 8. When working with proteins containing His-tags, it may be preferable to use a pH of about 8 to avoid protein insolubility or precipitation which may otherwise occur in lower pH buffered solutions.

Buffer exchange and concentration of the micellar solution may be achieved using suitable techniques and devices known in the art, *e.g*. using a Micro Bio-Spin ® column (Bio-Rad Laboratories) or a Vivaspin device (GE Healthcare).

The membrane protein is detected using a mass spectrometer comprising a nanoelectrospray ionisation source, a mass analyser and a detector. The mass spectrometer is preferably adapted to transmit and detect ions having mass-to-charge (*m*/*z*) ratios in the range of e.g. from about 100 *m*/*z* to about 32,000 *m*/*z.* Preferably, the mass spectrometer is operated under conditions suitable for maintaining and focusing large macromolecular ions. By way of illustration, and without limitation, the mass spectrometer may be a Synapt HDMS quadrupole-S2 ion-trap-IM-MS instrument, and more preferably a G2 instrument. The resolution provided by such instruments is particularly suited to resolving peaks generated from a complex comprising a membrane protein bound to a therapeutic agent or other ligand.

The nanoelectrospray ionisation source is used to vaporise the micellar solution. Nanoelectrospray ionisation is a technique well known in the art (see *e.g.* Wilm et al, Anal. Chem. 1996, 68, 1-8; and Wilm et al, Int. J. of Mass Spec. and Ion Proc. 1994, 132, 167-180). The use of nanoelectrospray ionisation allows ions, and in particular highly charged ions, to be generated directly from solution. The formation of highly charged ions may allow the detection of high mass complexes at relatively low mass-to-charge (*m*/*z*) ratios. The use of a nanoelectrospray ionisation is also desirable from the point of view of allowing a membrane protein complex, or subunits of a complex, to remain substantially intact. In performing a method of the present invention, it may be preferable to use a nanoflow capillary, *e.g*. a gold-coated nanoflow capillary, to vaporise the solution.

The micellar solution is vaporised under conditions such that the membrane protein is released from the micelle. The membrane protein may be released from the micelle as a result of collisions between the electrospray and the micelle which serve to disrupt the detergent assembly. Preferably, the vaporisation conditions are selected so that the membrane protein is released from the micelle substantially intact. Preferably, the conditions inside the mass spectrometer are selected to rapidly remove the micelle from the membrane protein. Ionisation of the membrane protein may occur during the step of vaporising and/or after release of the protein from the micelle. In some instances, portions of the membrane protein, *e*.*g*. hydrophilic/cytoplasmic domains, may become ionised prior to release of the protein from the micelle. Typically, ionisation of the membrane protein occurs during and/or after release of the protein from the micelle. In an embodiment, release and/or ionisation of the protein from the micelle occurs in a collision cell present within the mass spectrometer. Release and/or ionisation of the protein from the micelle may be achieved by adjusting acceleration voltages and/or pressures within the collision cell to remove the detergent while retaining the peaks of the membrane protein.

Mass spectrometer parameters may be optimised for maximal desolvation and detergent removal, while minimising protein activation. In particular, one or more of the following parameters may be optimised: collision voltage, cone voltage, collision gas pressure, collision gas type, and source pressure. Optimisation of parameters may be achieved by first setting the instrument parameters to relatively high activation settings for membrane proteins. Then iteratively, each of the aforementioned four parameters may be adjusted to produce resolved mass spectra while minimizing over-activation of the target protein.

In an embodiment, the mass spectrometer is operated under one or more of the following conditions: (i) a capillary voltage of from about 0.8 to about 2.2 kV, *e.g*. from about 1.0 to about 2.0 kV, *e.g*. from about 1.2 to about 1.8 kV; (ii) a cone voltage of from about 10 to about 240 V, *e.g.* from about 10 to about 50 V, *e.g.* from about 100 to about 200 V; (iii) a trap collision energy of from about 50 to about 240 V, *e.g*. from about 100 to about 220 V, e.g. from about 120 to about 200 V; (iv) a source temperature of from about 0 to about 50 °C , e.g. from about 10 to about 30 °C , *e.g*. about 20 °C; (v) a bias voltage of from about 20 to about 200 V, *e.g*. from about 60 to about 180 V, *e.g*. from about 80 to about 160 V; and (vi) a backing pressure of from about 1 to about 8 mBar, *e.g*. from about 3 to about 6 mBar, e.g. from about 4 to about 5 mBar. In a particular embodiment, the mass spectrometer is operated with a bias voltage of from about 30 to about 200 V, *e.g*. from about 60 to about 180 V, *e.g.* from about 80 to about 160 V. In a particular embodiment, the trap collision energy is from about 50 to about 200 V.

Preferably, minimal activation energy is used to liberate the membrane protein from the detergent micelle. In an embodiment, the laboratory frame energy is from about 500 to about 5000 electron volts, *e.g*. from about 500 to about 1500 electron volts. The term "laboratory frame energy" as used herein refers to the collision voltage multiplied by charge state of the membrane protein.

The ionised membrane protein is then resolved and detected and, if desired, further characterised. In particular, in embodiments in which the membrane protein is complexed with a therapeutic agent, ions in which the therapeutic agent is bound to the membrane protein or a fragment thereof can be detected directly using the mass spectrometer, rather than inferred indirectly from mass spectra of the separate components (therapeutic agent and protein). Moreover, where the micellar solution or the complex comprises one or more additional components, *e.g*. one or more components selected from lipids and nucleotides, the binding of one or more of said components to the membrane protein may be detected simultaneously. For instance, the method may comprise detecting a plurality of ions selected from the group consisting of ions containing the therapeutic agent bound to the membrane protein or a fragment thereof, ions containing one or more additional components (*e.g*. selected from lipid and nucleotides) bound to the membrane protein or a fragment thereof, and ions in which the therapeutic agent and one or more additional components are bound to the membrane protein or a fragment thereof. Thus, the present methods may be used to detect concomitant binding of the membrane protein with *e.g.* a therapeutic agent and one or more other species which compete for binding sites.

In a particular embodiment, the membrane protein is detected using ion mobility-mass spectrometry (IM-MS). In this case, the mass spectrometer may comprise an ion mobility cell to assess the folded state of membrane protein complexes. The use of IM-MS may allow the stoichiometry of ligand agent binding, and the overall effects of ligand binding on the dynamics, stabilities, oligomeric structures and conformations of membrane proteins, to be determined. For instance, the stoichiometry and oligomeric structure of complexes may be characterised by assessing mass differences. As a further example, dynamics, stabilities and conformations may be characterised by changes in charge states or by differences in arrival time distributions (*i.e*. ion mobility).

The following non-limiting Examples illustrate the present invention.

### Example 1

The activation energy required to achieve resolved peaks of *E. coli* ammonium channel C-terminally fused to green fluorescent protein (AmtB-GFP) was assessed for a variety of non-ionic detergents.

A green fluorescent protein (GFP) expression plasmid was constructed by subcloning the multiple cloning site region from XbaI (New England Biolabs) and BlpI (New England Biolabs) of pET23b (Novagen) into the backbone pET15b (Novagen). The resulting engineered vector was linearized by NdeI (New England Biolabs) and NheI (New England Biolabs) such that an Infusion cloning reaction (Clonetech) resulted in a TEV cleavable C-terminal fusion to superfolder GFP (subcloned from Gandhi et al, Protein Science 2011, 20, 313-326) followed by a 6x His-tag.

The AmtB and AQPZ genes were amplified by polymerase chain reaction (PCR) with Phusion high-fidelity DNA polymerase (New England Biolabs) from Ecoli genomic DNA with primers designed for an Infusion (Clonetech) reaction using the manufacturer's online tool. The PCR products were purified by agarose gel electrophoresis and extracted using the QIAquick Gel Extraction Kit (Qiagen, Valencia, CA). The PCR products were used in an Infusion cloning reaction according to a manufacturer protocol to generate AmtB-GFP and AQPZ-GFP. The final constructs were verified by DNA sequencing.

The AmtB-GFP and AQPZ-GFP plasmids were transformed into Ecoli BL21 (DE3) Gold (Agilent). Several colonies were inoculated into 50 mL LB Miller (5 g yeast extract, 10 g peptone from casein, and 10 g sodium chloride per litre) and grown overnight at 37 °C. One litre of LB in 2 L shaker flasks was inoculated with 7 mL of overnight culture and grown at 37 °C until the culture reached an OD600 between 0.6-0.8. Isopropyl β-D-1-thiogalactopyranoside (IPTG) was added to a final concentration of 0.5 mM and grown for 3 hours at 37 °C. Cells were harvested by centrifugation at 5,000 g for 10 minutes at 4 °C. The cell pellets were resuspended in water or buffer, pelleted by centrifugation at 5,000 g for 10 minutes at 4 °C frozen, and stored at -80 °C.

Cell pellets were thawed and resuspended at 20 mL per litre of culture in Buffer A (300 mM sodium chloride, and 20 mM TRIS, pH 7.4 at room temperature) supplemented with a complete protease inhibitor tablet (Roche) and 5 mM beta-mercaptoethanol (BME). Cell suspension was passed several times through an M-110 PS microfluidizer (Microfluidics) at 19,000 psi. Insoluble material was pelleted by centrifugation at 20,000 g for 25 minutes at 4 °C. Membranes were pelleted by centrifugation at 100,000 g for 2 hours at 4 °C. Membranes were resuspended in ice-cold Buffer B (100 mM sodium chloride, 10% glycerol, 5 mM BME, and 20 mM TRIS, pH 7.4 at room temperature), homogenized using a Potter-Elvehjem teflon pestle and glass tube, and used either directly or flash frozen in liquid nitrogen and stored at -80 °C.

AmtB-GFP was extracted from purified membranes (described above) with 1-2% (w/v) of the detergent of interest in Buffer B and incubated for one to three hours at room temperature or overnight at 4 °C. Insoluble material was pelleted by centrifugation at 20,000 g for 25 minutes at 4 °C. The clarified supernatant was loaded onto small Ni-NTA agarose (Qiagen) drip columns (Bio-spin Chromatography columns, Bio-Rad) and washed with several column volumes of Buffer C (200 mM sodium chloride, 20 mM imidazole, 5mM BME, 2x CMC detergent of interest, and 50 mM TRIS, pH 8.0 at room temperature). AmtB-GFP was eluted with two column volumes of Buffer D (100 mM sodium chloride, 250 mM Imidazole, 5mM BME, 2x CMC detergent of interest, and 50 mM TRIS, pH 8.0 at room temperature). Eluted protein was concentrated using a 100 kDa MWCO concentrator and buffer exchanged into MS Buffer (2x CMC detergent of interest and 200 mM Ammonium Acetate, pH 8.0 with Ammonium Hydroxide) using a centrifugal buffer exchange device (Micro Bio-Spin 6, Bio-Rad).

Buffer exchanged protein was analysed on a Q-TOF 2 mass spectrometer (Micromass, Manchester, UK) with a Z-spray source including modifications for protein complexes after introduction of complexes from gold-coated nanoflow capillaries.

The results of these experiments are presented in Table 1 below, which shows the mean charge state (Zavg) and the standard deviation (Zwidth) for each detergent tested.

**Table 1**

| **Detergent** | **Abb.** | **Experimental Mass** | **Zavg^{b}** | **Zwidth^{a}** |
|---|---|---|---|---|
| ***Glucosides*** | | | | |
| n-Octyl-β-D-glucopyranoside | OG | 214,499.59 ± 68.10 | 31.2 | 1.83 |
| n-Nonyl-β-D-glucopyranoside | NG | 214,495.08 ± 48.13 | 30.4 | 1.69 |

| ***Thioglucosides*** | | | | |
|---|---|---|---|---|
| n-Octyl-β-D-thioglucopyranoside | OTG | 214,286.14 ± 24.30 | 31.9 | 1.72 |

| ***Maltosides*** | | | | |
|---|---|---|---|---|
| n-Decyl-β-D-maltoside | DM | 214,424.76 ± 65.50 | 31.4 | 1.63 |
| n-Dodecyl-β-D-maltoside | DDM | 214,742.12 ± 89.54 | 30.4 | 1.66 |
| n-Undecyl-β-D-maltoside | UDM | 214,424.79 ± 49.23 | 30.3 | 1.84 |
| Cymal-5 | Cy5 | 214,453.24 ± 43.00 | 31.3 | 1.63 |
| Cymal-6 | Cy6 | 214,552.86 ± 50.01 | 30.5 | 1.82 |

| ***Thiomaltosides*** | | | | |
|---|---|---|---|---|
| n-Dodecyl-β-D-thiomaltopyranoside | DDTM | 215,973.74 ± 195.09 | 30.0 | 2.05 |

| ***Alkyl Glycosides*** | | | | |
|---|---|---|---|---|
| Octyl Glucose Neopentyl Glycol | OGNG | 214,560.48 ± 60.46 | 27.9 | 2.0 |

| ***Polyoxyethylene Glycols*** | | | | |
|---|---|---|---|---|
| Triton X-100 | TX100 | 214,537.99 ± 49.55 | 21.6 | 2.18 |
| C8E4 | C₈E₄ | 214,589.77 ± 86.60 | 21.3 | 1.61 |
| C8E5 | C₈E₅ | 214,872.76 ± 440.16 | 22.8 | 1.75 |
| C12E8 | C₁₂E₈ | 214,728.43 ± 107.62 | 25.8 | 1.8 |
| Anapoe-58 (Brij-58) | C₁₆E₂₀ | 214,598.44 ± 188.77 | 25.0 | 1.92 |

| | | | | |
|---|---|---|---|---|
| ^{a} Mass calculated using spectra collected with cone/collision voltages of 180/200 with the exception of C₁₂E₈ where 150/190 was used. The reported mass is the average mass along with the standard deviation. ^{b} Charge state envelopes were modelled with a Gaussian. | | | | |

As the data presented above demonstrate, the use of a polyoxyalkylene glycol detergent results in a significant lowering in the charge state (Zavg) of the membrane protein as compared with other detergents. Consequently, less activation energy is needed to liberate the membrane protein from the detergent micelle.

### Example 2

Mass spectra activation series for AmtB-GFP and Aquaporin Z (AQPZ) were obtained using a variety of non-ionic detergents.

AmtB-GFP and AQPZ-GFP were extracted from purified membranes in 2% OG in Buffer B and incubated overnight at 4 °C. Extracted membrane proteins were clarified by centrifugation at 20,000 g for 25 minutes at 4 °C. The clarified supernatant was filtered before loading onto a 5 mL HisTrap-HP column (GE Healthcare, Piscataway, NJ) equilibrated in Buffer E (200 mM sodium chloride, 10% glycerol, 20 mM imidazole, 0.025% DDM, and 50 mM TRIS, pH 7.4 at room temperature). After the clarified supernatant was loaded, the column was initially washed with 40-50 mL of Buffer E containing 1% OG instead of DDM followed by several column volumes of Buffer E until a steady baseline was reached. Protein was eluted with a linear gradient to 100% in two column volumes of Buffer F (100 mM sodium chloride, 10% glycerol, 500 mM Imidazole, 0.025% DDM, and 50 mM TRIS, pH 7.4 at room temperature). Peak fractions were pooled, supplemented with 5mM BME and His-tagged TEV protease, and dialyzed against Buffer G (150 mM sodium chloride, 10% glycerol, 20 mM Imidazole, 0.02% DDM, and 50 mM TRIS, pH 7.4 at room temperature) overnight at 4 °C. After overnight incubation at 4 °C, samples were filtered and passed back over a 5 mL HisTrap-HP column equilibrated in Buffer E. Flow through containing the untagged membrane protein was collected and concentrated using a 100 kDa MWCO concentrator. Concentrated protein was flash frozen in liquid nitrogen and stored at -80 °C.

Frozen samples were thawed on ice prior to detergent exchange over gel filtration. Samples were injected on a Superdex 200 10/300 (GE Healthcare) equilibrated in Buffer H (130 mM sodium chloride, 10% glycerol, and 50 mM TRIS, pH 7.4 at room temperature) supplemented with 0.5% of C8E4, 0.4% of NG, or 0.02% of DDM. Peak fractions were concentrated using a 100 kDa MWCO concentrator. Concentrated protein was either used directly or flash-frozen in liquid nitrogen and stored at -80 °C. Purified membrane proteins were buffer exchanged into MS Buffer (2x CMC detergent of interest and 200 mM Ammonium Acetate, pH 8.0 with Ammonium Hydroxide) using a centrifugal buffer exchange device (Micro Bio-Spin 6, Bio-Rad).

Buffer exchanged protein was analysed on a quadrupole ion mobility time-of-flight mass spectrometer (Synapt HDMS, Waters Corp.) modified with an 18 cm drift cell with radial RF confinement (RF amplitude 200 V) and a linear voltage gradient along the axis of ion transmission such that collision cross section (CCS) values could be determined directly.

Figure 1 depicts mass spectra activation series for various detergents on AmtB-GFP. Purified *E. coli* AmtB-GFP in n-Dodecyl-β-D-maltoside (DDM) was used as the starting material for detergent exchange. It can be seen that C₈E₅, a polyoxyethylene glycol detergent, reduced the average charge state of the trimeric complex similarly to others within this detergent family. It should also be noted that the trimeric complex has shifted from one centred at 7,000 m/z to 9,500 m/z corresponding roughly to a charge state reduction of eight.

Figure 2 show a mass spectra activation series for AmtB-GFP detergent exchanged into various polyoxyethylene glycol detergents, namely: (a) Triton X-100, (b) C₈E₄, (c) C₁₂E₈, and (d) C₁₆E₂₀ (Anapoe-58). It can be seen that the overall charge state of the membrane protein complex was reduced to an average charge state of +23 in these detergents compared to the average charge state of +31 in maltoside detergents.

Figure 3 shows a comparison of mass spectra for AQPZ and AmtB membrane protein complexes in three detergents: polyoxyethylene glycol C₈E₄ (C₈E₄), n-Nonyl-β-D-glucopyranoside (NG), and n-Dodecyl-β-D-maltoside (DDM). Mass spectra were acquired with instrument set to 10 volts for sample cone and 200 volts for collision voltage. It can be seen that for both protein complexes in C₈E₄, the charge state has not only been reduced but the mass spectra are mainly comprised of the expected oligomer complexes with no observable dissociation products. In stark contrast, DDM and NG mass spectra give rise to mainly dissociation products with moderate to weak intensities for the expected oligomer complex.

Figure 4 shows ion mobility mass spectrometry of AQPZ and AmtB membrane protein complexes in polyoxyethylene glycol C₈E₄ (C₈E₄), n-Nonyl-β-D-glucopyranoside (NG), and n-Dodecyl-β-D-maltoside (DDM). Both membrane protein complexes in C₈E₄ detergent enable the measurement of collisional cross section (CCS) values consistent with calculated CCS values from their respective crystal structure by the projection approximation approach around E_{LAB} values of <1500. The preservation of native structure is most likely due to the charge reducing behaviour of C₈E₄, as well as other polyoxyalkylene glycol detergents, which results in a two to five fold reduction in activation energy. In addition, under E_{LAB} values slightly greater than 1500, the CCS value increases consistent with partial unfolding. More importantly, gas-phase unfolding can be observed for these membrane complexes at E_{LAB} values of 1750 to 2250. These gas-phase unfolding transitions can be altered in the presence of a drug molecule that stabilises protein structure, thereby enabling the detection of drug molecules that stabilise protein structure. Protein complexes in DDM displayed all unfolded CCS values, even at the lowest energy to obtain resolved mass spectra. Although the detergent NG requires lower activation as compared to DDM, the experimental CCS indicates the complex is unfolded. Interestingly, AmtB in NG displayed gas-phase unfolding, but no CCS could be observed indicating native structure. These results highlight the application of polyoxyalkylene glycol detergents in not only preserving native structure but the ability to monitor gas-phase unfolding which is useful for identifying ligands (*e*.*g*. drugs) that bind and stabilise membrane protein structure.

### Example 3

A mass spectrometry approach was used to monitor the effects of individual lipid binding events on various membrane protein complexes anticipated to respond differently to the phospholipid environment. Three membrane protein complexes were selected to give a range of topologies, oligomeric states and anticipated selectivity towards phospholipids: (i) the pentameric mechanosensitive channel of large conductance (MscL) from *Mycobacterium tuberculosis* with two transmembrane helices (TMH) and an intimate relationship with phospholipids; (ii) the tetrameric water efflux channel Aquaporin Z (AQPZ) from *E. coli* with six TMH for which associated phospholipid or detergent molecules have been revealed in crystal structures and in related homologues; and (iii) the trimeric ammonia channel (AmtB) from *E. coli,* with eleven TMH involved in the transport of ammonia/ammonium, for which no phospholipid binding has previously been observed in crystal structures.

To study the membrane protein-lipid interactions by MS, the native state of the multimeric assemblies was maintained, devoid of detergent from the micelles required for their introduction, such that stabilisation by lipid binding could be deduced. This was previously not considered possible since the conditions needed to disrupt the micelle perturb the folded state of membrane complexes.

To assess individual lipid binding events, the following synthetic and natural phospholipid solutions were prepared and then added to protein complexes in detergent:
1,2-dinervonoyl-*sn*-glycero-3-phosphocholine (24:1 Cis) ("DNPC");
1,2-dierucoyl-*sn*-glycero-3-phosphocholine (22:1 Cis) ("DEPC");
1,2-dieicosenoyl-*sn*-glycero-3-phosphocholine (20:1 Cis) ("DIPC");
1,2-dioleoyl-*sn*-glycero-3-phosphocholine (18:1 Cis) ("DOPC");
1,2-dipalmitoleoyl-*sn*-glycero-3-phosphocholine (16:1 Cis) ("DPPC");
1,2-dimyristoleoyl-*sn*-glycero-3-phosphocholine (14:1 Cis) ("DMPC");
1-palmitoyl-2-oleoyl-*sn*-glycero-3-phosphocholine (16:0-18:1) ("PC");
1-palmitoyl-2-oleoyl-*sn*-glycero-3-phospho-L-serine (16:0-18:1) ("PS");
L-α-phosphatidylglycerol (*E*. *coli)* ("PG");
1-palmitoyl-2-oleoyl-*sn*-glycero-3-phosphate (16:0-18:1) ("PA");
L-α-phosphatidylethanolamine (*E*. *coli*) ("PE");
1,2-dioleoyl-*sn*-glycero-3-phospho-(1'-myo-inositol-3'-phosphate) (18:1) ("PI"); and Cardiolipin (*E*. *coli)* ("CDL").

Tetraethylene glycol monooctyl ether (C₈E₄) was employed as the detergent in these experiments, as lower average charge states were observed with rotationally averaged collision cross sections (CCS) for this detergent, providing optimal conditions for MS of intact membrane protein complexes.

IM-MS was used to resolve individual phospholipid-bound states of membrane proteins, in folded conformations, devoid of the complication of the lipid bilayer. Bound phospholipids were then ranked based on their ability to prevent gas-phase unfolding. Combining the IM-MS data for the three protein complexes with molecular dynamic (MD) simulations, probable lipid binding sites were then identified. The crystal structure of phosphatidylglycerol (PG), identified herein as significantly stabilising the ammonium channel, was then determined in this lipid environment. The 2.3Å resolution structure revealed bound lipids in one of the sites predicted by IM-MS. In addition, by comparison with previous structures, this resolution structure also revealed distinct conformational changes induced by phospholipid binding that reposition residues to interact with the phospholipid bilayer.

### Methods: Plasmid construction

Two expression plasmids were constructed first by subcloning the multiple cloning site region from XbaI and BlpI of pET23b (Novagen) into the backbone pET15b (Novagen). The resulting engineered vector was linearized by NdeI (New England Biolabs) and XhoI (New England Biolabs), gel purified (QIAquick Gel Extraction Kit, Qiagen), and used in subsequent InFusion cloning reactions (Clonetech) to generate a TEV protease cleavable C-terminal fusion to superfolder GFP (subcloned from Gandhi *et al, supra*) followed by a 6x His-tag or a TEV protease cleavable N-terminal fusion to maltose binding protein preceded by a secretion signal peptide (pelB) and 10x His-tag subcloned from Hilf et al (Nature, 2008, 452, 375-379). The resulting N- and C-terminal fusion vectors were linearized with NheI (New England Biolabs) and XhoI or NdeI and NheI, gel purified, and used in subsequent InFusion cloning reactions. AmtB (residues 26-428), AQPZ and MscL genes were amplified by polymerase chain reaction (PCR) with Phusion high-fidelity DNA polymerase (New England Biolabs) from prepared *E*. *coli* BL21 (DE3) genomic DNA (Qiagen) or template plasmid DNA with primers designed for an Infusion cloning reaction using the manufacturer's online tool. The PCR products were purified by agarose gel electrophoresis and extracted using the QIAquick gel extraction kit (Qiagen). The PCR products and linearized vectors (described above) were used in InFusion cloning reactions (Clonetech) to generate MBP-AmtB, AQPZ-GFP and MscL-GFP. All constructs were verified by DNA sequencing.

### Methods: Generation of E. coli BL21(DE3) ΔmscL::Kan^{R} strain

Homologous recombination mediated chromosomal disruption of *mscL* was performed using the Gene Doctoring method (see Lee et al, BMC Microbiology, 2009, 9, 252). Briefly, 900 base pairs flanking the 5' and 3' ends of the *mscL* gene were subcloned into the pDOC-K plasmid resulting in these regions flanking a kanamycin resistance (Kan^{R}) cassette. This plasmid and the recombineering plasmid, pACBSCE, were transformed into *E. coli* BL21 (DE3) Gold (Agilent). Homologous recombination was carried out by induction of λ-Red recombinase system and SceI endonuclease with arabinose. Recombinants harbouring gene disruption with Kan^{R} were selected on kanamycin and sucrose, and verified by colony PCR. Calcium chloride competent cells were made as described by Drew et al (Nat. Methods, 2006, 3, 303-313).

### Methods: Membrane protein expression

MBP-AmtB and AQPZ-GFP plasmids were transformed into *E. coli* BL21 (DE3) Gold (Agilent). MscL-GFP was transformed into *E. coli* BL21(DE3) Δ*mscL::Kan^{R}* (described above). Several colonies were inoculated into 50 mL LB Miller (5 g yeast extract, 10 g peptone from casein, and 10 g sodium chloride per litre) and grown overnight at 37 °C. One litre of LB in 2 L shaker flasks was inoculated with 7 mL of overnight culture and grown at 37 °C until the culture reached an OD₆₀₀ between 0.6-0.8. Isopropyl β-D-1-thiogalactopyranoside (IPTG) was added to the culture at a final concentration of 0.5 mM and cells grown for 3 hours at 37 °C. Cells were harvested by centrifugation at 5,000 g for 10 minutes at 4 °C. Cell pellets were resuspended in buffer, pelleted by centrifugation at 5,000 g for 10 minutes at 4 °C and stored at -80 °C.

### Methods: Purification of membranes

Membranes were purified as described by Newby et al (Nat. Protoc., 2009, 4, 619-637) with minor modifications. Cell pellets were thawed and resuspended at 20 mL per litre of culture in Buffer A (300 mM sodium chloride, and 20 mM 2-amino-2-hydroxymethyl-propane-1,3-diol (Tris), pH 7.4 at room temperature) supplemented with a complete protease inhibitor tablet (Roche) and 5 mM beta-mercaptoethanol (BME). The cell suspension was passed several times through an M-110 PS microfluidizer (Microfluidics) at 19,000 psi. Insoluble material was pelleted by centrifugation at 20,000 g for 25 minutes at 4 °C. Membranes were pelleted by centrifugation at 100,000 g for 2 hours at 4 °C. Membranes were resuspended in ice-cold Buffer B (100 mM sodium chloride, 20% glycerol, 5 mM BME, and 20 mM Tris, pH 7.4 at room temperature), homogenized using a Potter-Elvehjem Teflon pestle and glass tube. For MscL-GFP, glycerol was omitted from Buffer B. Resuspended membranes were used either directly or flash frozen in liquid nitrogen and stored at -80 °C.

### Methods: Purification of membrane protein fusions

MBP-AmtB and AQPZ-GFP were extracted from purified membranes in Buffer B supplemented with 1.5-2% OG and incubated with gentle agitation overnight at 4 °C. MscL was extracted from purified membranes with glycerol-free Buffer B supplemented with 1% OGNG. Extracted membrane proteins were clarified by centrifugation at 20,000 g for 25 minutes at 4 °C. Supernatant was filtered before loading onto a 5 mL HisTrap-HP column (GE Healthcare, Piscataway, NJ) equilibrated in Buffer E (200 mM sodium chloride, 10% glycerol, 20 mM imidazole, 0.025% DDM, and 50 mM Tris, pH 7.4 at room temperature). After the clarified supernatant was loaded, the column was initially washed with 40-50 mL of DDM-free Buffer E supplemented with 1% OG for MBPAmtB and AQPZ-GFP, and 0.5% OGNG for MscL-GFP. Membrane proteins were then exchanged into several column volumes of Buffer E until a steady baseline was reached. Membrane protein fusions were eluted with a linear gradient to 100% in two column volumes of Buffer F (100 mM sodium chloride, 10% glycerol, 500 mM imidazole, 0.025% DDM, and 50 mM Tris, pH 7.4 at room temperature). Peak fractions were pooled, supplemented with 5 mM BME and His-tagged TEV protease, and dialyzed against Buffer G (150 mM sodium chloride, 10% glycerol, 20 mM imidazole, 0.02% DDM, and 50 mM Tris, pH 7.4 at room temperature) overnight at 4 °C. After overnight incubation, samples were filtered and passed back over a 5 mL HisTrap-HP column equilibrated in Buffer E. Flow through containing the untagged membrane protein was collected and concentrated using a 100 kDa MWCO concentrator. Tag-removed membrane proteins with fusions to the C-terminus contained additional protein sequence ASGENLYFQ, or GAS for N-terminally tagged proteins, resulting from the TEV protease recognition sequence and cloning restriction site. Concentrated protein was either used immediately or flash-frozen in liquid nitrogen and stored at -80 °C. Protein concentration was measured using a Biomate UV detector with the following calculated extinction coefficients of 1.57, 1.484, and 0.265 g/L•cm⁻¹ for AmtB, AQPZ, and MscL, respectively.

### Methods: Preparation of membrane proteins for native mass spectroscopy

Either flash frozen samples were thawed on ice or fresh samples were detergent exchanged by gel filtration chromatography. Membrane protein samples were injected onto a Superdex 200 GL 10/300 (GE Healthcare) column equilibrated in Buffer H (130 mM sodium chloride, 10% glycerol, and 50 mM Tris, pH 7.4 at room temperature) supplemented with either 0.5% of C₈E₄, 0.4% of NG, 0.116% OGNG, or 0.02% of DDM. Peak fractions containing the detergent-exchanged membrane protein complex were concentrated. A 50 kDa MWCO concentrator was used to concentrate samples in the detergent C₈E₄ and 100 kDa MWCO concentrator used on membrane protein complexes in all other detergents. Concentrated proteins were either used directly or flash frozen in liquid nitrogen and stored at -80 °C. Notably, no observable difference in mass spectra quality was found after a single freeze-thaw of membrane proteins throughout the purification regime. Purified membrane proteins were buffer exchanged into MS Buffer (two times the CMC of detergent of interest and 200 mM ammonium acetate, pH 7.2-8.0 with ammonium hydroxide) using a centrifugal buffer exchange device (Micro Bio-Spin 6, Bio-Rad). Membrane proteins detergent exchanged into MS Buffer supplemented with C8E4 or OGNG could be flash frozen without compromising mass spectra quality unlike MS Buffer supplemented with NG or DDM.

### Methods: Preparation and titration of phospholipids

Phospholipids were purchased from Avanti (Avanti Polar Lipids Inc., Alabama, USA) and prepared at stock concentrations of 10 mg/ml in 200 mM ammonium acetate pH 8.0. The membrane protein complex to phospholipid to detergent ratio (P:L:D) was optimized for each membrane protein complex to achieve resolved mass spectral peaks of bound phospholipid throughout the gas-phase unfolding series and consistency of nanoelectrospray. Empirically optimized conditions to maintain sufficient mass spectral quality and phospholipid binding are shown in Table 2 below. Phospholipid preparation was added to buffer exchanged membrane protein complexes followed by equilibration at room temperature for 10-30 minutes before MS analysis. Notably, a significant increase in bound phospholipid with longer incubation times was not observed with MS.

**Table 2: Protein to phospholipid to detergent ratios (P:L:D) for each membrane protein, and number of resolvable phospholipids bound**

| **Protein** | **Phospholipid** | **Protein (µM)** | **Phospholipid (µM)** | **Detergent (µM)** | **Ratio (P:L:D)** | **# Bound** |
|---|---|---|---|---|---|---|
| AQPZ | CDL | 7 | 70 | 16300 | 1:10:2300 | 1 |
| | PE | 7 | 348 | - | 1:50:2300 | 1 |
| | PG | 9 | 87 | - | 1:10:1757 | 3 |
| | PS | 9 | 383 | - | 1:40:1757 | 3 |
| | PC | 7 | 132 | - | 1:18:2300 | 3 |
| | PA | 7 | 449 | - | 1:61:2300 | 2 |
| AmtB | CDL | 3 | 140 | 16300 | 1:45:5197 | 1 |
| | PE | 2 | 70 | - | 1:44:10395 | 2 |
| | PG | 4 | 99 | - | 1:28:4559 | 4 |
| | PS | 4 | 92 | - | 1:26:4559 | 3 |
| | PC | 4 | 95 | - | 1:27:4559 | 3 |
| | PA | 2 | 69 | - | 1:44:10395 | 3 |
| MscL | CDL | 17 | 175 | 8157 | 1:10:480 | 1 |
| | PE | 17 | 70 | - | 1:4:4.80 | 1 |
| | PG | 17 | 131 | - | 1:8:480 | 2 |
| | PS | 17 | 128 | - | 1:7:480 | 2 |
| | PC | 17 | 132 | - | 1:7:480 | 3 |
| | PA | 17 | 143 | - | 1:8:480 | 1 |
| | PI | 17 | 52 | - | 1:6:480 | 4 |
| | DNPC | 24 | 79 | - | 1:3:350 | 2 |
| | DEPC | 24 | 56 | - | 1:2:350 | 2 |
| | DIPC | 24 | 89 | - | 1:4:350 | 2 |
| | DOPC | 24 | 95 | - | 1:4:350 | 2 |
| | DPPC | 24 | 137 | - | 1:6:350 | 2 |
| | DMPC | 24 | 74 | - | 1:3:350 | 2 |

### Methods: Mass spectrometry

Mass spectrometer settings were initially set to values for membrane proteins for a modified Q-TOF 2 mass spectrometer (Micromass, Manchester, UK) with a Z-spray source. Typical instrument values were 5-7 µbar for source pressure, 1.5-1.8 kV for capillary voltage, 150-190 V for cone voltage, 1-10 V for extraction voltage, 180-200 V for collision voltage, argon for collision gas, and 0.2-0.3 MPa for collision gas pressure.

### Methods: Ion mobility mass spectrometry

Ion mobility measurements were performed on a modified Synapt G1 HDMS instrument with the travelling-wave ion mobility cell replaced by an 18-cm drift cell with radial RF ion confinement and a linear voltage gradient to direct ions along the axis of transmission to the time-of-flight (TOF) mass analyser. Ion mobility mass spectrometer was typically set to a source pressure of 5-7 mBar, capillary voltage of 1.4-1.7 kV, capillary nanoflow of 0.03-0.2 mBar and argon as collision gas with flow rate set to 5-8 mL/min (-6.6-6.7 e-2 mBar). Collision voltage ranged from 2-240 V with measurements taken at 5 V and 2 V steps for monitoring gas-phase unfolding of membrane and soluble protein complexes, respectively. The sample and extraction cone and trap bias voltages, quadrupole profile, and collision gas pressure were optimized for maximal ion intensity of the target membrane protein complex. Helium was the drift cell gas and set to a pressure of ∼1.6-1.8 Torr (40 ml/min flow rate). Pusher time, pulse width and TDC inhibit were set to 180, 7 and 7 µs, respectively. The ion guide/source (300 ms⁻¹, 10 V), trap (300 ms⁻¹, 0.2 V) and transfer (100 ms⁻¹, 10 V) travelling wave velocities and wave heights were kept constant. The mobility release time was set at 200 ps with a trap voltage of 30 V and extract voltage of 10 V. Pressure and temperature of the ion mobility cell was measured directly. Ion drift time (td) was determined as described in Bush et al (Analytical Chemistry, 2010, 82, 9557-9565).

### Methods: Collision cross section calculations

The Projection Approximation (PA) calculation within a modified version of MOBCAL (see Shvartsburg et al, Chem. Phys. Letters, 1996, 261, 86-91; Mesleh et al, J. Phys. Chem., 1996, 100, 16082-16086; and Baldwin et al, Structure, 2011, 19, 1855-1863) was used to calculate the Ω_{PA} from respective crystal structure coordinates. CCS values were corrected Ω_{*Corr*'} by the correction factor of 1.14 and missing residues between the protein construct (*M_{Exp}*) and crystal structure (*M_{PDB}*) (see Benesch et al, Curr. Opin. Struc. Biol., 2011, 21, 641-649; and Hall et al, Structure, 2012, 20, 1596-1609). This correction has been shown to give values that correlate with known values to ± 3%.

### Methods: Gas-phase unfolding data analysis and modelling

Mass spectra and ion mobility data were analysed using software written in Python programming language with a graphical user interface constructed using wxPython. Briefly, MS-only data collected on a Q-TOF 2 instrument were smoothed with MassLynx software prior to being imported and linearized at a step size of one in m/z. Theoretical mass spectra were predicted following equations described by Stengel et al (Chem. Biol., 2012, 19, 599-607). Mass, average charge state, width of charge state distribution, resolution, and concentration were fitted to experimental data with least squares regression of the pseudo-χ² function.

Ion mobility data were imported using a custom script provided by Waters at an *m*/*z* resolution of four in drift data and one in mass spectra for fitting. Mass spectra were smoothed and theoretical mass spectra were fitted as outlined above. A standardized integration window was employed for extracting arrival time distributions (ATD) using a resolution (R = *m*/*z* ÷ *Δm*/*z*) of 1000 and a height cut-off of 25% for the modelled Gaussian to the respective *m*/*z* peak. To generate gas-phase unfolding plots, ATDs were extracted for the given *m*/*z* peak using the standardized integration window followed by normalization. To convert to a CCS axis, arrival time was directly converted to CCS using equations above. This procedure generates gas-phase unfolding data and plotted using Matplotlib. All ion mobility mass spectra are shown in linear scale.

### Methods: AmtB crystallisation and structure determination

AmtB was purified as described for native mass spectrometry experiments with one minor modification. AmtB was detergent exchanged into Buffer H containing 0.5% C₈H₄ using a Superdex 200 10/300 gel filtration column (GE Healthcare). Peak fractions of AmtB from gel filtration were pooled and concentrated to -15 mg/mL using a 50 kDa MWCO concentrator (Millipore). AmtB was titrated with a ten-fold molar excess of phosphotidylglycerol prior to crystallization. Crystals of AmtB were grown at 20 °C in hanging drop plates with crystallization solution 15% PEG 4000, 0.8M potassium formate, and 0.1 M sodium acetate pH 4.6. Hexagonal plate crystals appeared after one month.

Single crystals were mounted with CrystalCap HT Cryoloops (Hampton Research, Aliso Viejo, CA) before being flash frozen. Data were collected at Diamond Light Source beam line l04. Diffraction data were processed with XDS in apparent space group P622. Cell content analysis suggested two molecules in the asymmetric unit cell and attempts to obtain phases from molecular replacement using PHASER with pdb 1U7G resulted in placement of only one molecule with a missing layer of protein molecules. Twinning was suspected and the data were processed into lower symmetry space groups until molecular replacement could correctly place all protein molecules in the asymmetric unit cell, which turned out to be space group C222. Data were submitted to a twin detection server for twinning analysis that indicated partial twinning with <|L|> = 0.463 (untwinned = 0.5, perfectly twinned = 0.375) and <L2> = 0.291 (untwinned = 0.333, perfectly twinned = 0.200). Despite the presence of twinning, initial phases were found by molecular replacement using PHASER with pdb 1U7G placing six molecules in the asymmetric unit cell, and followed automated model building using PHENIX and manual model building using COOT. All model refinement was done with REFMAC in twin mode. Omit maps were generated first by deleting coordinates of the ligand prior to refinement. Refinement with twinning applied resulted in a 10% drop in both R_{work} and R_{free}, consistent with twinning. Figures were generated with PyMOL.

### Results

MscL was interrogated first and well-resolved mass spectral peaks could be obtained corresponding to populations of MscL in complex with one to five phospholipids (see Figure 5b). To investigate the effects of individual phospholipid binding on stability, collision-induced unfolding was applied. At low collision energies (70 V), the complex is in its native state. At an intermediate collision voltage (130 V), +12/+13 charge states show an increase in CCS from the trend line calculated for the native state to highly extended conformers (Figure 5b). All species, apo and one to four phospholipid-bound forms, are measured within the same experiment enabling a direct comparison, and show an increasing population of native pentameric states as a function of phospholipid binding.

To extract quantitative values for the effects of phospholipid binding on unfolding pathways, an established equilibrium unfolding model, used extensively in solution studies involving chemical denaturation, was applied. Plotting CCS against collision voltage reveals unfolding trajectories from the native state, through intermediates, to extended forms while maintaining their oligomeric state (Figure 5c). By comparing the *apo* form of the protein with lipid bound states, the average stabilisation was calculated by fitting the unfolding pathway for the most abundant charge state, identifying four distinct intermediate states. The average stabilisation computed for bound phospholipids allowed seven different phospholipids to be compared and ranked, as well as synthetic phosphatidylcholine (PC) lipids whose chain length varied from C14 to C22, to determine which is most able to prevent unfolding of MscL. Despite the difference in these phospholipids, each was found to be similarly stabilising (Figure 5d), consistent with a previous fluorescence study with engineered Trp residues (see Powl et al, Biochemistry, 2003, 42, 14306-14317). In addition, it was found that MscL bound avidly to phosphatidylinositol (PI) conferring a large increase in stability when binding multiple lipids, greater than all other phospholipids investigated. To locate potential phospholipid binding sites, molecular dynamics (MD) simulations in POPC bilayers and filtered candidate models using CCS measurements were used. An even distribution of phospholipids was predicted over the surface of the transmembrane region of MscL (see Figure 5e).

A similar procedure was applied to AQPZ, revealing well-resolved lipid binding and a CCS in agreement with that of the X-ray structure of AQPZ (see Figure 6a). Monitoring unfolding trajectories for lipid-bound states of AQPZ showed similar transition points between the different conformations that were largely independent of the bound phospholipid implying that the phospholipids investigated had similar effects on resistance to unfolding (see Figure 6b). As successive lipids are added, from one to three, stabilisation was increased via regular increments consistent with a linear, cumulative effect. The only exception to this was cardiolipin (CDL) which resulted in a substantial increase in protein stability compared to all other phospholipids. Using MD and CCS measurements, phospholipids were located primarily to subunit interfaces centred in the membrane, suggesting that the POPC tails fill in surface grooves and plug a hydrophobic pocket between AQPZ subunits (see Figure 6c).

By contrast with MscL and AQPZ, few lipids were found to bind AmtB, and only low populations of CDL molecules were co-transported into the gas phase. Binding of phosphatidic acid (PA) or phosphatidylserine (PS), conversely, was extensive. By measuring resistance to unfolding, it was found that binding of PA and PS was not correlated with significant stabilisation of the channel, in contrast to MscL and AQPZ (see Figure 7b). MD simulations and CCS measurements localised POPC phospholipids to the interface between subunits as well as to several smaller patches on individual subunits. Distinct from PA or PS, addition of phosphatidylglycerol (PG) resulted in a dramatic increase in stabilisation with cumulative binding linearly increasing protein stability (see Figures 7a and 7b). These results reveal a defined preference for PG-like head groups that are stabilising and demonstrate selective phospholipid binding events that lead to an enhanced stability for AmtB.

To understand the origin of PG induced stability, AmtB was crystallised in the presence of PG phospholipids. A screen produced X-ray-grade crystals and led to structure determination at 2.3Å resolution. The structure of AmtB is formed by three protein subunits each compromising 11 TMH with two trimers located in the asymmetric unit cell. Eight PG molecules were resolved into electron density on the periplasmic side of AmtB. Two of the PG molecules are located in similar positions at the subunit interface with headgroups not in contact with the protein, suggesting a possible generic phospholipid binding site. The crystallographic PG molecules at the subunit interface align within the ensemble of phospholipids predicted by MD simulation and filtered by CCS measurements, consistent with a non-selective binding site. The overall structure is largely similar to the 20 previously determined structures in the Protein Data Bank (PDB) determined from detergent only environments. Major differences arise from a distinct conformational change in a loop, residues 70-81, forming a specific phospholipid binding site. PG forms hydrogen bonds to N72 and E70 and a water bridge to N79. F75 flips downward to interact with a lipid tail of PG. In addition, several residues are repositioned to interact with the phospholipid bilayer. L81 and I78 shift downward into the hydrophobic environment of the lipid bilayer. Significantly W80 moves almost 4Å (Cα to Cα), its sidechain being rotated 160° compared to other structures. This rotation relocates W80 sidechains from a protein environment into one that is ideally positioned to interact with headgroups of phospholipids, consistent with their alignment along the lipid bilayer interface. In a structure of AmtB in complex with GlnK, an octyl glucoside molecule is bound in a similar position to that occupied by PG. However, the protein conformation in this region of the AmtB:GlnK complex is essentially identical to all other structures in the PDB, implying that the conformational changes observed here are most likely induced upon specific phospholipid binding

Further data are presented in Figures 9 and 10. Figure 9 shows mass spectra and ion mobility analysis of AQPZ tetramer from *E. coli* bound to up to four phosphatidylcholine phospholipid molecules revealing well resolved peaks in both dimensions, as well as the achievement of native-like state in the gas phase. Figure 10a shows a four state gas-phase unfolding series of the pentameric MscL in its apo (i) and phosphatidylinositol (PI) (ii) bound state. Figure 10b shows average stabilisation of the MscL protein complex bound to different phospholipid forms and numbers reveals differences in protein complex stabilisation upon small molecule binding.

Overall, by resolving individual phospholipid bound states of membrane protein complexes and obtaining quantitative values lipid-binding interactions are able to be ranked for their effects on stability. For MscL non-selective lipid binding, without regard to particular headgroup or chain length, is sufficient to increase stability, in accord with the ability of the channel to bind and respond promiscuously to lipid composition. Major cumulative effects on stability are observed upon binding of PI, a key component of the *Mycobacterium* membrane involved in MscL mechanosensitivity. For AQPZ, multiple lipid binding events were observed for all phospholipids. Binding a single phospholipid resulted in equal stabilisation, implying similar binding modes, which is in line with MD simulations in phospholipid bilayers across the aquaporin family and with two-dimensional crystals of AQPZ. The only exception to this was CDL which stabilises AQPZ significantly, indicating a potential role in plugging hydrophobic gaps in subunit interfaces predicted. The finding that AmtB responds only weakly to the majority of phospholipids is consistent with its extensive transmembrane structure and inherent stability devoid of the phospholipid bilayer. Its unexpected selectivity for binding PG lipids identified by IM-MS led to the crystal structure in complex with phospholipid. The major effects on stability can therefore be rationalized by binding of PG to subunit interfaces and the conformational changes observed upon integration of PG lipids. Taken together, these results support the notion that individual phospholipids, devoid of the bilayer properties, can have a profound effect on membrane protein stability. The most highly stabilising lipids likely induce conformational change as shown here for PG binding to AmtB. More generally, separating lipids that bind, from those that induce stability, gives a powerful distinction not only for downstream structural biology but also for understanding membrane protein function in the biological membrane.

Thus, the present methods may enable an important distinction to be made between small molecules that merely bind to a membrane protein and those that also stabilise the protein, consequently revealing the effects of lipid, drug or cofactor binding on membrane protein structure and function.

### Example 4

Using similar procedures, mass spectra were obtained for intact Acriflavine resistance protein B (AcrB) from *Escherichia coli* using a mixture of detergents. Spectra were collected from a 200 mM ammonium acetate solution pH 8.0, containing a detergent mixture of 0.5 % Triton X-100 and 1.5 % C₈E₄. The results of this experiment are presented in Figure 11.

## Claims

1. A method of detecting a membrane protein by mass spectrometry, wherein the method comprises:
(a) providing a solution comprising a detergent micelle in which said membrane protein is contained, wherein said solution contains a polyoxyalkylene glycol detergent having a molecular weight of at least 300 Da;
(b) providing a mass spectrometer comprising a nanoelectrospray ionisation source, a mass analyser and a detector;
(c) vaporising the solution using the nanoelectrospray ionisation source under conditions such that the membrane protein is released from the micelle;
(d) ionising the membrane protein;
(e) resolving the ionised membrane protein using the mass analyser; and
(f) detecting the resolved membrane protein using the detector.

2. A method according to claim 1, wherein the membrane protein is an integral membrane protein, *e*.*g*. a G protein-coupled receptor, a membrane transporter, a drug efflux pump, an ATP-binding cassette transporter or a proton driven transporter.

3. A method according to claim 1, wherein the membrane protein is selected from EmrE, LmrP, MscL, BtuCD, BtuC₂D₂, LmrCD, MacB, MexB, P-gp, MsbA, NorM, KirBac3.1, AmtB and Aquaporin Z.

4. A method according to any preceding claim, wherein the membrane protein is in the form of a complex with one or more ligands, *e.g.* one or more ligands selected from therapeutic agents, lipids and nucleosides.

5. A method according to claim 4, wherein the membrane protein is in the form of a complex with a therapeutic agent, *e*.*g*. a drug, *e.g.* a non-polymeric organic compound having a molecular weight of less than 1000 Daltons.

6. A method according to any preceding claim, wherein the detergent is present in the solution at a concentration which is greater than or equal to the critical micelle concentration of the detergent in said solution.

7. A method according to any preceding claim, wherein the solution is an aqueous solution.

8. A method according to any preceding claim, wherein the molar ratio of the detergent to the membrane protein in the solution is from about 10:1 to about 150:1.

9. A method according to any preceding claim, wherein the membrane protein is released from the micelle substantially intact.

10. A method according to any preceding claim, wherein the mass spectrometer is operated under one or more of the following conditions: (i) the capillary voltage of the nanoelectrospray ionisation source is from about 0.8 to about 2.2 kV; (ii) the cone voltage of the nanoelectrospray ionisation source is from about 10 to about 240 V; (iii) the trap collision energy is from about 50 to about 240 V; (iv) the source temperature is from about 0 to about 50 °C; (v) the bias voltage is from about 20 to about 200 V; and (vi) the backing pressure is from about 1 to about 8 mBar.

11. A method according to any preceding claim, wherein the membrane protein is detected by ion mobility-mass spectrometry.

12. A method according to any preceding claim, wherein the polyoxyalkylene glycol detergent has a molecular weight of from 300 Da to 1000 Da, *e.g.* from 300 Da to 600 Da or from 500 Da to 1000 Da.

13. A method according to any preceding claim, wherein the polyoxyalkylene glycol detergent is a polyoxyethylene glycol detergent, *e*.*g*. tetraethylene glycol monooctyl ether ("C₈E₄"), pentaethylene glycol monooctyl ether ("C₈E₅"), pentaethylene glycol monodecyl ether ("C₁₀E₅"), polyoxyethylene (8) dodecyl ether ("C₁₂E₈"), polyoxyethylene (20) cetyl ether ("C₁₆E₂₀") or polyethylene glycol *tert*-octylphenyl ether (*t*-oct-C₆H₄-(OCH₂CH₂)ₓOH, where x is 9-10).

14. Use of a solution comprising a detergent micelle in which a complex is contained, wherein the complex comprises a membrane protein bound to one or more ligands, and wherein the solution comprises a polyoxyalkylene glycol detergent having a molecular weight of at least 300 Da, for the detection of said complex by mass spectrometry.

## Patentansprüche

1. Verfahren zum Detektieren eines Membranproteins durch Massenspektrometrie, wobei das Verfahren Folgendes beinhaltet:
(a) Bereitstellen einer Lösung, die eine Detergens-Mizelle umfasst, in der das genannte Membranprotein enthalten ist, wobei die genannte Lösung ein Polyoxyalkylenglykol-Detergens mit einer Molekülmasse von wenigstens 300 Da enthält;
(b) Bereitstellen eines Massenspektrometers, das eine Nanoelektrospray-Ionisationsquelle, einen Massenanalysator und einen Detektor umfasst;
(c) Verdampfen der Lösung unter Verwendung der Nanoelektrospray-Ionisationsquelle unter solchen Bedingungen, dass das Membranprotein von der Mizelle freigesetzt wird;
(d) Ionisieren des Membranproteins;
(e) Auflösen des ionisierten Membranproteins mittels des Massenanalysators; und
(f) Detektieren des aufgelösten Membranproteins mit dem Detektor.

2. Verfahren nach Anspruch 1, wobei das Membranprotein ein integrales Membranprotein ist, z.B. ein G-Protein-gekoppelter Rezeptor, ein Membrantransporter, eine Arzneimitteleffluxpumpe, ein ATP-bindender Kassettentransporter oder ein protonengetriebener Transporter.

3. Verfahren nach Anspruch 1, wobei das Membranprotein ausgewählt ist aus EmrE, LmrP, MscL, BtuCD, BtuC₂D₂, LmrCD, MacB, MexB, P-gp, MsbA, NorM, KirBac3.1, AmtB und Aquaporin Z.

4. Verfahren nach einem vorherigen Anspruch, wobei das Membranprotein in Form eines Komplexes mit einem oder mehreren Liganden, z.B. einem oder mehreren Liganden, ausgewählt aus therapeutischen Mitteln, Lipiden und Nucleosiden, vorliegt.

5. Verfahren nach Anspruch 4, wobei das Membranprotein in Form eines Komplexes mit einem therapeutischen Mittel, z.B. einem Arzneimittel, z.B. einer nicht-polymeren organischen Verbindung mit einer Molekülmasse von weniger als 1000 Dalton, vorliegt.

6. Verfahren nach einem vorherigen Anspruch, wobei das Detergens in der Lösung in einer Konzentration vorliegt, die gleich der oder größer als die kritische(n) Mizellenkonzentration des Detergens in der genannten Lösung ist.

7. Verfahren nach einem vorherigen Anspruch, wobei die Lösung eine wässrige Lösung ist.

8. Verfahren nach einem vorherigen Anspruch, wobei das Molverhältnis zwischen Detergens und Membranprotein in der Lösung etwa 10:1 bis etwa 150:1 beträgt.

9. Verfahren nach einem vorherigen Anspruch, wobei das Membranprotein von der Mizelle im Wesentlichen intakt freigesetzt wird.

10. Verfahren nach einem vorherigen Anspruch, wobei das Massenspektrometer unter einer oder mehreren der folgenden Bedingungen betrieben wird: (i) die Kapillarspannung der Nanoelektrospray-Ionisationsquelle beträgt etwa 0,8 bis etwa 2,2 kV; (ii) die Kegelspannung der Nanoelektrospray-Ionisationsquelle beträgt etwa 10 bis etwa 240 V; (iii) die Fallenkollisionsenergie beträgt etwa 50 bis etwa 240 V; (iv) die Ausgangstemperatur beträgt etwa 0 bis etwa 50°C; (v) die Vorspannung beträgt etwa 20 bis etwa 200 V; und (vi) der Gegendruck beträgt etwa 1 bis etwa 8 mBar.

11. Verfahren nach einem vorherigen Anspruch, wobei das Membranprotein durch Ionenmobilitäts-Massenspektrometrie detektiert wird.

12. Verfahren nach einem vorherigen Anspruch, wobei das Polyoxyalkylenglykol-Detergens eine Molekülmasse von 300 Da bis 1000 Da, z.B. von 300 Da bis 600 Da oder von 500 Da bis 1000 Da, hat.

13. Verfahren nach einem vorherigen Anspruch, wobei das Polyoxyalkylenglykol-Detergens ein Polyoxyethylenglykol-Detergens ist, z.B. Tetraethylenglykolmonooctylether ("C₈E₄"), Pentaethylenglykolmonooctylether ("C₈E₅"), Pentaethylenglykolmonodecylether ("C₁₀E₅"), Polyoxyethylen-(8)-Dodecylether ("C₁₂E₈"), Polyoxyethylen-(20)-cetylether ("C₁₆E₂₀") oder Polyethylenglykol-tert-octylphenylether (t-oct-C₆H₄-(OCH₂CH₂)ₓOH, wobei x 9-10 ist).

14. Verwendung einer Lösung, die eine Detergens-Mizelle umfasst, in der ein Komplex enthalten ist, wobei der Komplex ein an einen oder mehrere Liganden gebundenes Membranprotein umfasst und wobei die Lösung ein Polyoxyalkylenglykol-Detergens mit einer Molekülmasse von wenigstens 300 Da umfasst, zum Detektieren des genannten Komplexes durch Massenspektrometrie.

## Revendications

1. Procédé de détection d'une protéine membranaire par spectrométrie de masse, dans lequel le procédé comprend :
(a) la fourniture d'une solution comprenant une micelle de détergent dans laquelle ladite protéine membranaire est contenue, dans lequel ladite solution contient un détergent de polyoxyalkylène glycol ayant un poids moléculaire d'au moins 300 Da ;
(b) la fourniture d'un spectromètre de masse comprenant une source d'ionisation par nano-électronébulisation, un analyseur de masse et un détecteur ;
(c) la vaporisation de la solution en utilisant la source d'ionisation par nano-électronébulisation dans des conditions telles que la protéine membranaire est libérée de la micelle ;
(d) l'ionisation de la protéine membranaire ;
(e) la résolution de la protéine membranaire ionisée en utilisant l'analyseur de masse ;
(f) la détection de la protéine membranaire résolue en utilisant le détecteur.

2. Procédé selon la revendication 1, dans lequel la protéine membranaire est une protéine membranaire intégrale, par exemple un récepteur couplé à une protéine G, un transporteur membranaire, une pompe à efflux médicamenteuse, un transporteur à cassette de liaison à l'ATP, ou un transporteur à entraînement protonique.

3. Procédé selon la revendication 1, dans lequel la protéine membranaire est choisie parmi EmrE, LmrP, MscL, BtuCD, BtuC₂D₂, LmrCD, MacB, MexB, P-gp, MsbA, NorM, KirBac3.1, AmtB et l'Aquaporine Z.

4. Procédé selon l'une quelconque des revendications précédentes, dans lequel la protéine membranaire est sous la forme d'un complexe avec un ou plusieurs ligands, par exemple un ou plusieurs ligands choisis parmi des agents thérapeutiques, des lipides et des nucléosides.

5. Procédé selon la revendication 4, dans lequel la protéine membranaire est sous la forme d'un complexe avec un agent thérapeutique, par exemple un médicament, par exemple un composé organique non polymérique ayant un poids moléculaire inférieur à 1 000 daltons.

6. Procédé selon l'une quelconque des revendications précédentes, dans lequel le détergent est présent dans la solution à une concentration qui est supérieure ou égale à la concentration micellaire critique du détergent dans ladite solution.

7. Procédé selon l'une quelconque des revendications précédentes, dans lequel la solution est une solution aqueuse.

8. Procédé selon l'une quelconque des revendications précédentes, dans lequel le rapport molaire du détergent et de la protéine membranaire dans la solution est d'environ 10/1 à environ 150/1.

9. Procédé selon l'une quelconque des revendications précédentes, dans lequel la protéine membranaire est substantiellement intacte quand elle est libérée de la micelle.

10. Procédé selon l'une quelconque des revendications précédentes, dans lequel le spectromètre de masse est amené à fonctionner dans une ou plusieurs des conditions suivantes : (i) la tension capillaire de la source d'ionisation par nano-électronébulisation est d'environ 0,8 à environ 2,2 kV ; (ii) la tension de cône de la source d'ionisation par nano-électronébulisation est d'environ 10 à environ 240 V ; (iii) l'énergie de collision du piège est d'environ 50 à environ 240 V ; (iv) la température de la source est d'environ 0 à environ 50 °C ; (v) la tension de polarisation est d'environ 20 à environ 200 V ; et
(vi) la pression de refoulement est d'environ 1 à environ 8 mBar.

11. Procédé selon l'une quelconque des revendications précédentes, dans lequel la protéine membranaire est détectée par spectrométrie de masse à mobilité ionique.

12. Procédé selon l'une quelconque des revendications précédentes, dans lequel le détergent de polyoxyalkylène glycol a un poids moléculaire de 300 Da à 1 000 Da, par exemple de 300 Da à 600 Da ou de 500 Da à 1000 Da.

13. Procédé selon l'une quelconque des revendications précédentes, dans lequel le détergent de polyoxyalkylène glycol est un détergent de polyoxyéthylène glycol, par exemple un éther mono-octylique de tétraéthylène glycol (« C₈E₄ »), un éther mono-octylique de pentaéthylène glycol (« C₈E₅ »), un éther monodécylique de pentaéthylène glycol (« C₁₀E₅ »), un éther dodécylique de polyoxyéthylène (8) (« C₁₂Eₛ »), un éther cétylique de polyoxyéthylène (20) (« C₁₆E₂₀ ») ou un éther tert-octylphénylique de polyéthylène glycol (*t*-oct-C₆H₄-(OCH₂CH₂)ₓOH, où X est 9 à 10).

14. Utilisation d'une solution comprenant une micelle de détergent dans laquelle un complexe est contenu, dans laquelle le complexe comprend une protéine membranaire liée à un ou plusieurs ligands, et dans laquelle la solution comprend un détergent de polyoxyalkylène glycol ayant un poids moléculaire d'au moins 300 Da, pour la détection dudit complexe par spectrométrie de masse.
